# EUROPEAN PATENT SPECIFICATION

(11) **EP 2 688 938 B1**
(45) Date of publication and mention of the grant of the patent: **16.10.2019**
(21) Application number: 11713113.6
(22) Date of filing: 21.03.2011
(51) Int. Cl.: C08G 77/50, C08G 77/60, A01N 25/10, A01N 25/30, A61K 8/81, A61K 8/894, C05G 3/00, C05G 3/06, C07F 7/08, C08F 220/28, C08F 220/56, C09D 133/14, C09D 133/24, C08G 77/48

(54) **ORGANOMODIFIED CARBOSILOXANE MONOMERS CONTAINING COMPOSITIONS AND USES THEREOF**
ORGANOMODIFIZIERTE CARBOSILOXANMONOMERE ENTHALTENDE ZUSAMMENSETZUNGEN UND IHRE VERWENDUNG
COMPOSITIONS CONTENANT DES MONOMÈRES CARBOSILOXANES ORGANO-MODIFIÉS ET LEURS UTILISATIONS

(43) Date of publication of application: 29.01.2014
(73) Proprietor: Momentive Performance Materials Inc., Waterford, NY 12188 (US)
(72) Inventor: SAXENA, Anubhav, Bangalore 560027 (IN); SENTHILKUMAR, Umapathy, Bangalore 560066 (IN); LEWIS, Kenrick, M., Flushing, NY 11358 (US)
(74) Representative: Gille Hrabal
(86) International application number: PCT/US2011/029164
(87) International publication number: WO 2012/128751

(56) References cited:
- EP-A1- 2 258 412
- EP-A2- 0 367 381
- WO-A2-2009/085298
- WO-A2-2009/085299
- WO-A2-2010/038242
- GB-A- 2 137 635
- US-A- 5 347 028
- US-A1- 2004 040 554
- US-A1- 2011 009 519
- US-A1- 2011 009 658
- US-B1- 6 391 999
- EFIMOV YU T ET AL: "SYNTHESIS OF ORGANOSILICON DERIVATIVES OF ACRYLIC ACIDS", JOURNAL OF GENERAL CHEMISTRY USSR, CONSULTANTS BUREAU, NEW YORK, NY, US, vol. 61, no. 10 PART 02, 1 October 1991 (1991-10-01), pages 2083-2091, XP000289683, ISSN: 0022-1279
- M. ISHIKAWA ET AL.: "Photochemically Generated Silicon-Carbon Double-Bonded Intermediates", J. ORGANOMETAL. CHEM., vol. 149, 1978, XP002666626,
- GREBER G ET AL: "UEBER OLIGOMERE SILICIUMVERBINDUNGEN MIT FUNKTIONELLEN GRUPPEN 7. MITT.: UEBER DIE HERSTELLUNG UND POLYMERISATION VON VINYL- UND ALLYLPOLYSILMETHYLENEN", MAKROMOLEKULARE CHEMIE, HUTHIG UND WEPF VERLAG, BASEL, CH, vol. 52, 1 January 1962 (1962-01-01), pages 184-198, XP001160714, ISSN: 0025-116X, DOI: 10.1002/MACP.1962.020520116
- B.A. OMOTOWA ET AL.: "Preparation and Characterization of Nonpolar Fluorinated Carbosilane Dendrimers by APcI Mass Spectrometry and Small-Angle X-ray Scattering", J. AM. CHEM. SOC., vol. 121, 1999, pages 11130-11138, XP002666627,

## Description

### FIELD OF THE INVENTION

The present invention relates to a composition comprising novel polymerizable organo modified carbosiloxane monomer that exhibits improved hydrolytic stability under acidic, basic and neutral pH conditions in comparison to the corresponding conventional siloxane monomer. More particularly the present invention relates to improved compositions comprising hydrolytic stability at the pH of 6.5, 7.0 and 7.5.

### BACKGROUND OF THE INVENTION

Any publications or references discussed herein are presented to describe the background of the invention and to provide additional detail regarding its practice. Nothing herein is to be construed as an admission that the inventors are not entitled to antedate such disclosure by virtue of prior invention.

Siloxane based hydrogels are used for variety of applications in the area of health care, personal care, agriculture, coatings and home care because of their water absorbing and high oxygen permeable nature. WO 2010/038242 A2 is directed at mono-functional hydrophilic silicone-containing monomers having a polyether with a branched linking group, which can be applied in the provision of homo-polymers and copolymers and which are useful in making water-absorbing silicone-hydrogel films for contact lens applications. The document is also directed at the production of such monomers and polymers. In contrast to the monomers according to the present invention containing a polyether group, the monomers disclosed therein do not incorporate the polyether group as a part of the monomer backbone, but as a branch-like substituent. Further, the monomers applicable in the formation of polymers and hydrogels disclosed in WO 2010/038242 A2 do not have carbosilane units of the general structure Si-alkylene-Si, but only siloxane units. However these silicone hydrogels show poor hydrolytic stability under acidic or basic pH conditions. This effect is predominant when the siloxane chain length is too small. Especially, the trisiloxane-based compounds are well known to undergo rapid hydrolytic cleavage under acidic or basic pH conditions. The instant invention discloses siloxane compositions that are hydrolytically more stable and can be used to form hydrolytically stable silicone hydrogel copolymer that can be potentially used for various applications, including those cited above.

Carbomer is a generic name for synthetic high molecular weight polymers of acrylic acid used as thickening, dispersing, suspending and emulsifying agents in pharmaceuticals and cosmetics. They may be homopolymers of acrylic acid, as well as copolymers with methacrylic acid and other acrylates.

### SUMMARY OF THE INVENTION

An object of the present invention is to provide a polymerizable organo-modified carbosiloxane composition comprising a silicone monomer having the following general formulae (I):

(R¹R²R³)Si-Y¹-[Si(R⁴R⁵)(Y²)]ₐ-Si(R⁶R⁷)-Z (I)

wherein a is 0 to about 100; Y¹ is a substituted or unsubstituted divalent alkyl linking group of 1 to about 10 carbon atoms;
Y² is oxygen;
R¹, R², R³, R⁴, R⁵, R⁶, and R⁷ is independently selected from the group consisting of monovalent aliphatic, cycloaliphatic or aromatic hydrocarbon groups of 1 to about 10 carbons and halogenated hydrocarbon groups of 1 to about 10 carbons Z has the following general formula (II)

   -R¹¹-B-X (II)

   wherein R¹¹ is a branched, divalent alkyl linking group having up to about 20 carbon atoms or a fluorinated hydrocarbon, a aralkyl or arylalkyl group, and B is a divalent hydrophilic functional moiety consisting of aliphatic, cycloaliphatic or aromatic hydrocarbons containing hetero atoms
   and X a polymerizable group
   selected from the group consisting of substituted or unsubstituted unsaturated aliphatic or aromatic hydrocarbons, acrylates and methacrylates, or
   wherein R¹¹ is a linear, divalent alkyl linking group having up to about 20 carbon atoms or a fluorinated hydrocarbon, an aralkyl or arylalkyl group, B is a divalent hydrophilic or hydrophobic functional moiety selected from the group of the following divalent moieties

   -O-(C₂H₄O)ₚ-(C₃H₆O)_{q}-(C₄H₈O)ᵣ-

   wherein p and q are independently 0 to about 100; r is 0 to about 50 and (p +q + r) is greater than 0, 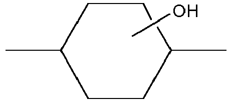 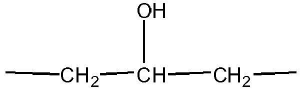 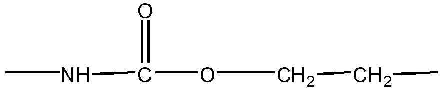 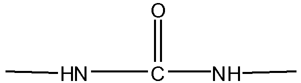 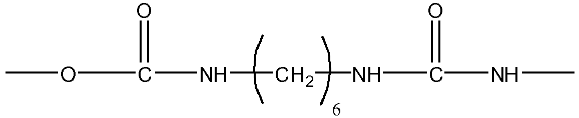 and X is a polymerizable group selected from the group consisting of substituted or unsubstituted unsaturated aliphatic or aromatic hydrocarbons, acrylates and methacrylates

Another object of the present invention is to provide a silicone composition comprising homo and copolymers derived from the monomers described herein.

Another object of the present invention is to provide a carbomer-containing composition exhibiting a high level of dispersion of its carbomer component, useful in the preparation of various personal care products and cosmetics. The inventive composition and formulations made there from are efficiently and effectively realized employing conventional equipment and standard manufacturing practices.

Another embodiment of the present invention is directed to a process for producing the described silicone monomers comprising chemically reacting a silicone-containing compound having the general formula shown below

(R¹R²R³)Si-Y¹-[Si(R⁴R⁵)(Y²)]ₐ-Si(R⁶R⁷)-H (IV)

wherein a is up to 100;
Y¹ is a substituted or unsubstituted divalent alkyl linking group of 1 to 10 carbon atoms; Y² is oxygen. R¹ to R⁷ is independently selected from the group consisting of monovalent aliphatic, cycloaliphatic or aromatic or halogenated hydrocarbon groups of 1 to about 10 carbons; with terminally unsaturated group having the general formula as shown below (V)

   R¹⁵-B-M (V)

   wherein R¹⁵ is abranched unsaturated alkyl group having up to about 20 carbon atoms, B is a divalent hydrophilic functional moiety consisting of aliphatic, cycloaliphatic or aromatic hydrocarbons and substituted or unsubstituted, saturated and unsaturated aliphatic, cycloaliphatic or aromatic hydrocarbons containing hetero atoms, wherein B comprises functionalities selected from the group consisting of alkyl, alcohol, ether, ester, amide, amine, acid and its salts, cyano, thio, urethane, urea, carbonate, carbamate, sulfonate, sulphonamide, phosphate and combinations thereof; M is selected from the group consisting of hydroxyl, halogen, epoxy and carboxylic acid group.

The reaction products are called carbosiloxanes. Once the functionalized carbosiloxane is produced it is reacted with an alkylacryloyl compound having the general formula (VI). 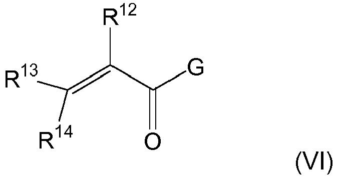 wherein G is selected from the group consisting of a halogen, hydroxyl and alkyloxy having 1 to about 10 carbon atoms and R¹², R¹³ and R¹⁴ is independently selected from the group consisting of hydrogen, substituted saturated monovalent hydrocarbons having 1 to about 20 carbons and unsubstituted saturated monovalent hydrocarbons having 1 to about 20 carbonsto produce said silicone monomer having the general formulae (I). The reaction of the functionalized carbosiloxane with alkylacryloyl compound having the general formula (VI) can be carried out in the presence of a tertiary amine base or basic ion-exchange resin (IER) or azeotrope-forming solvent or reactant. The azeotrope-forming solvent can be selected from hexane, heptane and toluene and the reactant such as methylmethacrylate under the inert reaction conditions.

### DETAILED DESCRIPTION OF THE INVENTION

In accordance with the present invention, compositions comprising polymerizable group selected from the group consisting of substituted or unsubstituted unsaturated aliphatic or aromatic hydrocarbons, acrylates and methacrylates functionalized carbosiloxane monomers that are non-bulky and show improved hydrolysis resistance are provided. Carbosiloxane monomers of the present invention showed improved hydrolysis resistance under acidic and basic pH conditions in comparison to the corresponding conventional siloxane monomers. Cured compositions produced from these monomers showed better oxygen permeability, and surface wettability and lower modulus in comparison to compositions/films of the corresponding conventional siloxane monomers.

As used herein, "homopolymers" are polymers made from the same repeating monomer and 'copolymers" are polymers wherein the polymer contains at least two structurally different monomers. Notations such as (meth)acrylate denote monomer with either acrylate or methacrylate functionality.

Also, as used in the specification and including the appended claims, the singular forms "a," "an," and "the" include the plural, and reference to a particular numerical value includes at least that particular value, unless the context clearly dictates otherwise.

All methods described herein may be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

As used herein, "comprising," "including," "containing," "characterized by," and grammatical equivalents thereof are inclusive or openended terms that do not exclude additional, unrecited elements or method steps, but will also be understood to include the more restrictive terms "consisting of" and "consisting essentially of."

Compositions comprising (meth)acrylate functionalized silicone monomers/polymers of the present invention, products made from these compositions as well as their preparation are further described in the sections below.

In the present invention, the monomers disclosed have a carbosilane linkage, -Si-(CH₂)ₙ-Si-, which makes it possible to produce compositions having hydrolytically stable (hydrolysis resistance) monomers and polymers. In particular, the present invention is directed to compositions comprising linear mono-acrylate functional organo-modified carbosiloxane compounds useful as a hydrolytically stable silicone monomer. The mono-acrylate functional carbosiloxane monomers of the present invention have the general structure shown in formula (I):

(R¹R²R³)Si-Y¹-[Si(R⁴R⁵)(Y²)]ₐ-Si(R⁶R⁷)-Z (I).

One of the preferred variants of the formula (I) of the present invention is the mono acrylate functional monomer having the general formula as shown below. 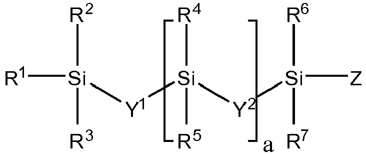 wherein a is 0 to 100; Y¹ is a substituted or unsubstituted divalent alkyl linking group of 1 to 10 carbon atoms, is oxygen. R¹ to R⁷ is independently selected from the group consisting of monovalent aliphatic, cycloaliphatic or aromatic hydrocarbon groups of 1 to about 10 carbons and halogenated hydrocarbon groups of 1 to about 10 carbons.

Z in the above structure has the general formula (II) shown below

-R¹¹-B-X (II)

wherein R¹¹ is a linear or branched, divalent alkyl linking group having about 0 to about 20 carbon atoms.
B in general formula (II) is a divalent hydrophilic functional moiety selected from the group consisting of aliphatic, cycloaliphatic or aromatic hydrocarbons containing hetero atoms in case R¹¹ is branched, or a divalent hydrophilic or hydrophobic functional moiety selected from the group of the following divalent moieties

-O-(C₂H₄O)ₚ-(C₃H₆O)_{q}-(C₄H₈O)ᵣ-

wherein p and q are independently 0 to about 100; r is 0 to about 50 and (p +q + r) is greater than 0, 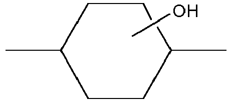 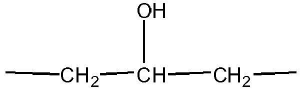 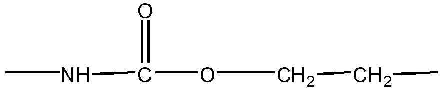 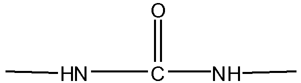 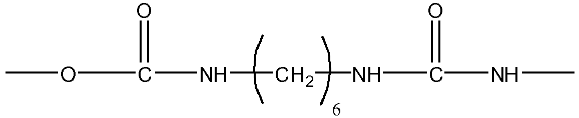 in case R¹¹ is linear; Preferably, B comprises functionalities such as alcohol, ether, esters, amides, amines, acids and its salts, cyano, thio, urethane, urea, sulfonates, sulphonamides, phosphates and their combinations.

In particular, some of the representative functionalities for B are shown below.

-O-(C₂H₄O)ₚ-(C₃H₆O)_{q}-(C₄H₈O)ᵣ-

Polyether
wherein p and q are independently 0 to about 100; r is 0 to about 50 and (p +q + r) is greater than 0. 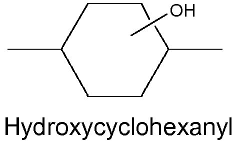 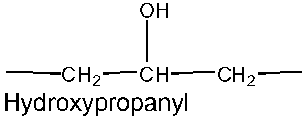 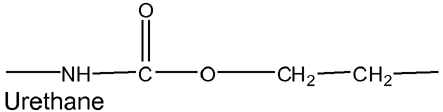 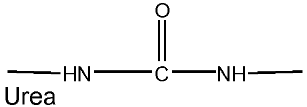 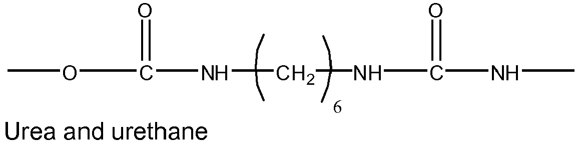 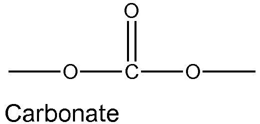 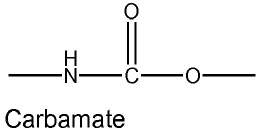 X is a polymerizable group having the general formula (III) 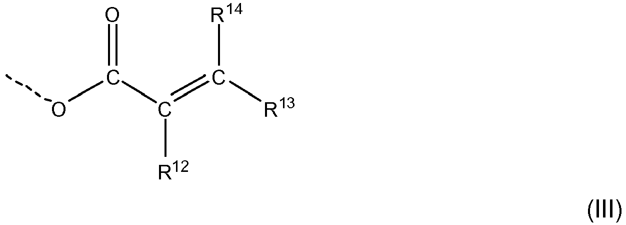 wherein R¹² to R¹⁴ is hydrogen or a substituted or unsubstituted saturated monovalent hydrocarbon group of 1 to about 20 carbons.

The present invention is also directed to compositions comprising polymers formed by the reaction products of the carbosiloxane monomers provided herein. These polymers may be homopolymers of one of the monomers of the present invention or copolymers of two structurally different silicone monomers of the present invention, and/or copolymers of one or more silicone monomers of the present invention and at least one other hydrophilic unsaturated organic monomers suitable for use in silicone hydrogels, with preferred non-limiting examples of such being N,N-dimethylacrylamide, 2-hydroxy-ethylmethacrylate (HEMA), 2-hydroxy ethylacrylate (HEA), N-vinylpyrrolidone, and methacrylic acid. In such copolymers, the ratio of the silicone monomers of the present invention to the other hydrophilic unsaturated organic monomers is from about 1:100 to about 100:1 and preferably from about 20:80 to about 90:10 and more preferably from about 30:70 to about 80:20.

The unsaturated organic monomers and the carbosiloxane monomers of this invention are mutually miscible and form homogeneous mixtures. The use of compatibilizing solvents is not necessary. The carbosiloxane monomers of this invention are also either water-soluble or water-dispersible. Water-soluble carbosiloxane monomers are miscible with water in all proportions to yield homogeneous solutions. Water-dispersible carbosiloxane monomers do not dissolve completely in water. Cloudiness, haze, colloid formation and similar visible signs of heterogeneity in the aqueous mixture are indicative of dispersion rather than solution. Both water solubility and water dispersibility are desirable features of the carbosiloxane monomers of the instant invention. When the carbosiloxane monomers contain a methacrylated ethoxylated polyether segment, water dispersibility is observed when the polyether content is less than about 60 weight percent of the total molecular weight, and water solubility when the polyether segment is greater than about 60 weight percent.

Prior to forming compositions comprising polymers, the polymers are formed by mixing the desired monomers and the resulting mixture is polymerized and cured to form transparent thin films by known thermal techniques using free radical or cationic or anionic initiators and UV cure techniques using photoinitiators in the presence of crosslinking agents. The monomers added to the reaction mixture to form the polymers may be monomers or prepolymers. A "prepolymer" is a reaction intermediate polymer of medium molecular weight having polymerizable groups. Thus, it is understood that the terms "silicone-containing monomers", "carbosiloxane monomers" and "hydrophilic monomers" include prepolymers.

One preferred variant of carbosiloxane monomer from structure (I) of the present invention has the following formula 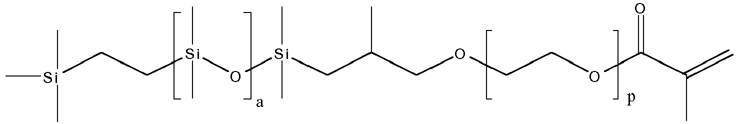 wherein B is a divalent polyether as shown in the representative example with p being up to about 100, preferably 2 to about 15, more preferably about 8, and q and r equal to 0; Y¹ is a divalent alkyl-linking group of about 1 to 10 carbons, preferably 1 to about 5 carbons, more preferably about 2 carbons. Y² is oxygen and X is polymerizable methacrylate group, a is 0 to about 100, more preferably 0 to 20 inclusive, and even more preferably 1. Each of the R groups in the general monomer structure (I) is a monovalent alkyl-linking group of 1 to 10 carbons, preferably a methyl group.

Another preferred variant of carbosiloxane monomer from structure (I) of the present invention has the following formula 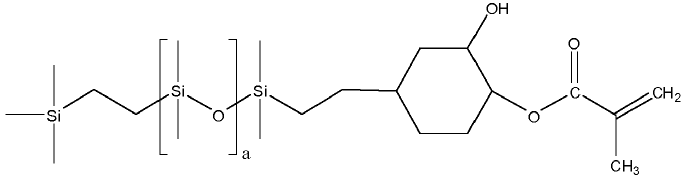 wherein B is a divalent hydroxyl containing cycloaliphatic ring; X is polymerizable methacrylate group, a is up to about 100, more preferably up to about 20 inclusive, and even more preferably 1. Y¹ is a divalent alkyl-linking group of about 1 to 10 carbons, preferably 1 to about 5 carbons, more preferably about 2 carbons. Y² is oxygen. Each of the R groups in the general monomer structure (I) is a monovalent alkyl-linking group, preferably a methyl group.

Another embodiment of the present invention is directed to a process for producing the described carbosiloxane monomers comprising chemically reacting a carbosiloxane compound with SiH functionality having the general formula shown below

(R¹R²R³)Si-Y¹-[Si(R⁴R⁵)(Y²)]ₐ-Si(R⁶R⁷)-H (IV)

wherein a is 0 to 100; Y¹ is a substituted or unsubstituted divalent alkyl linking group of 1 to 10 carbon atoms, and Y² is oxygen. R¹ to R⁷ is independently selected from the group consisting of monovalent aliphatic, cycloaliphatic or aromatic hydrocarbon groups of 1 to about 10 carbons and halogenated hydrocarbon groups of 1 to about 10 carbons. Once produced, a compound of general formula (IV) is reacted with a terminally unsaturated compound having the general formula as shown below (V)

R¹⁵-B-M (V)

wherein R¹⁵ is a branched unsaturated alkyl group having up to about 20 carbon atoms, B is a divalent hydrophilic functional moiety consisting of aliphatic, cycloaliphatic or aromatic hydrocarbons and substituted or unsubstituted, saturated and unsaturated aliphatic, cycloaliphatic or aromatic hydrocarbons containing hetero atoms; B comprises functionalities such as alcohols, ethers, esters, amides, amines, acids and its salts, cyano, thio, urethane, urea, sulfonates, sulphonamides, phosphates and their combinations. M is selected from hydroxyl or halogen or epoxy or carboxylic acid group.

Once the functionalized carbosiloxane is produced, it is reacted with an alkylacryloyl compound having the general formula (VI). 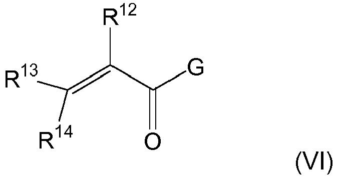 wherein G is selected from the group consisting of a halogen or hydroxyl or alkyloxy having 1 to 10 carbon atoms. R¹² to R¹⁴ are selected from hydrogen or a substituted or unsubstituted saturated monovalent hydrocarbon group of 1 to about 20 carbons to produce said silicone monomer. The reaction of the functionalized carbosiloxane with alkylacryloyl compound having the general formula (VI) can be carried out in the presence of a tertiary amine base or basic ion-exchange resin (IER) or azeotrope forming solvent or reactant. The azeotrope forming solvent can be selected from hexane, heptane, toluene and the reactant such as methylmethacrylate under the inert reaction conditions.

Once the monomers and polymers are produced as discussed above, they can be mixed with other monomers or polymers that are the same or different to produce the compositions of the present invention. These compositions have been found to have properties that are favorable for use in the cosmetic and medical industries. Some examples and discussions of the different uses for the compositions are provided herein below.

### USES FOR THE COMPOSITIONS OF THE PRESENT INVENTION:

### I. Health care:

The silicone hydrogel copolymers are gaining importance in the health care industry for various applications such as contact lens, wound management and drug delivery applications because of their improved oxygen permeability compared to conventional hydrogels. One of the key challenges in health care is that the material used for these applications should be dimensionally and chemically stable and should not undergo hydrolytic degradation and leach out degradation products under the conditions of use, such as sterilization and storage in aqueous media containing buffers. The carbosiloxane monomer of the instant invention shows improved hydrolytic stability over the conventional siloxane monomer, which enables it to produce silicone hydrogel copolymers with improved hydrolytic stability for health-care related applications, further details of which are shown in the following paragraphs. Also, its mono-functional polymerizable group (mono-acrylate or mono-methacrylate functionality) affords better control to produce silicone hydrogel copolymers with desired modulus depending upon the end applications.

### A. Contact lens:

The advantages of using the mono-functional carbosiloxane monomer of the instant invention for contact lens application are numerous. For example, (1) the presence of carbosiloxane linkage, -Si-(CH₂)ₙ-Si-, in the siloxane monomer composition of the present invention can help to produce soft hydrogel contact lens materials with improved hydrolytic stability. That means the silicone hydrogel contact lenses produced are hydrolytically stable and will not change in their chemical composition and physical dimensions when they are subjected to usage conditions, for e.g., in the eye or in the disinfecting solution or during sterilization. The contact lens material of the instant invention is also resilient. When the term resilient is used herein it is meant that after the lenses have been deformed the lenses will return quickly to their original shape. (2) The monomers of the instant invention, containing hydrophilic side chains, are compatible with hydrogel co-monomers in the entire range of compositions without employing any solvent or compatibilizing agents. The copolymer films obtained with various hydrogel comonomers and the mixtures thereof are transparent, water absorbing with inherently wettable surface. (3) Furthermore, the human cornea requires about 2×10⁻⁶ cm³/(sec. cm² atm.) of oxygen through the contact lens for better eye health as reported by Hill and Fatt (American Journal of Optometry and Archives of the American Academy of Optometry, Vol. 47, p. 50, 1970). The silicone-hydrogel contact lens materials made from the monomer of the instant invention allows better transmission of oxygen through itself to supply the necessary oxygen requirements of the human cornea. (4) The monomer of the instant invention can be used to produce hydrogel copolymer with the desired lower modulus to improve the comfort of the lens wearers. (5) The contact lens material can be produced with the monomer of the present instant invention employing the cure techniques (for eg. thermal and actinic radiation cure methods) known in the art with out employing solvent or compatibilizing agents.

### B. Wound management devices:

Hydrogels with high water content and oxygen permeability are needed to produce the wound-management devices (for eg. transdermal patches, wound healing patches, wound dressing patches). The water present in the wound-dressing material helps to provide a moist environment for better comfort and painless wound management. Oxygen also plays an important role in wound healing and the lack of oxygen transport in the wound dressing material has been identified as one of the most common issues affecting the wound healing process (Bok Y. Lee "The Wound Management Manual", McGraw-Hill, New York,2005, p.44). Oxygen transport to the wound through hemoglobin is an important process for wound healing. However, the damaged tissue in the wound can act as a barrier to hemoglobin leading to localized hypoxia at the wound site. Damaged tissue is generally hypoxic due to the large consumption of oxygen by cells. Leukocytes consume oxygen to produce infection-fighting oxidants. In addition, fibroblasts and endothelial cells also require oxygen for wound healing. Therefore, the only way of oxygen transport to the exterior part of the wound is from the atmosphere and this requires the wound dressing to have good oxygen permeability for better healing of the wounds. The monomer of the instant invention having hydrophilic moieties such as polyethers or other hydrophilic moieties can provide silicone hydrogels that are not only hydrolytically stable, but also water absorbing and highly oxygen permeable making them a suitable wound dressing material for various wound management applications. Hydrophilic silicone compositions of the present invention can also provide an excellent moist environment for better comfort and painless wound management. The silicone composition of current invention can be used as a potential carrier or device for various active ingredients and controlled release of those actives under different conditions (for example, pH, pressure, temperature, chemical reaction).

### C. Targeted and controlled delivery of bio-actives:

The ease of administration of drugs and the large surface area for absorption makes the gastro intestinal (GI) tract as the most popular route for drug delivery. Currently, several hydrogels are being investigated as potential devices for site-specific drug delivery for effective therapy. For example, biocompatible hydrogels are used as colon-specific drug delivery systems for the treatment of helicobacter pylori infection in peptic ulcer disease. These biocompatible hydrogels are designed to be highly swollen and to degrade in the presence of colonic enzymes or micro flora, providing colon-specific drug delivery. Additionally, biocompatible hydrogels protect insulin in the harsh, acidic environment of the stomach before releasing the drug in the small intestine. The hydrophilic silicone compositions of the present invention can be used to produce biocompatible silicone hydrogel-based drug delivery devices to release the drug locally to specific sites in the GI tract. The hydrophilic silicone compositions of this invention can also be used for other biocompatible drug delivery applications such as puncta plugs, ophtha coils, retinal implants, transdermal patches, wound healing patches and transdermal iontophoresis.

### D. Transdermal delivery:

The silicone compositions of the present invention can be used for the transdermal drug delivery applications. Drug delivery to the skin has been generally used to treat skin diseases or for disinfections of the skin. In recent years, transdermal route for the delivery of drugs has been investigated. Swollen soft hydrogels are explored to deliver the drugs for long period of duration and these hydrogels can be easily removed from the skin without pain and improves comfort for the patients. Current research in this field is now focused on iontophoresis and electroporation. Hydrogel-based formulations are being explored for electrically assisted delivery using transdermal iontophoresis for enhanced permeation of products such as, hormones and nicotine.

### E. Subcutaneous Delivery:

The silicone compositions of the present invention can be used for the subcutaneous drug delivery applications also. Among the various possible therapeutic applications of hydrogels, the most substantial application is implantable therapeutic devices. Hydrogel formulations having high water content, environmentally similar to biological tissue, make them relatively biocompatible and proposed for subcutaneous delivery of drugs. For example, hydrogels can be used for delivery of proteins and peptides. Currently, implantable hydrogels are leading towards the development of biodegradable systems, which do not require surgical removal of the drug-loaded implants.

Other health care applications include scaffolds for tissue engineering, anti-microbial devices, anti-bacterial devices, antifouling coatings, and anti-fungal devices.

### II. Pesticides-Agriculture, Horticulture. Turf, Ornamental and Forestry:

Many pesticide applications require the addition of an adjuvant to the spray mixture to provide wetting and spreading on foliar surfaces. Often that adjuvant is a surfactant, which can perform a variety of functions, such as increasing spray droplet retention on difficult to wet leaf surfaces, enhanced spreading to improve spray coverage, or to provide penetration of the herbicide into the plant cuticle. These adjuvants are provided either as a tank-side additive or used as a component in pesticide formulations. Typical uses for pesticides include agricultural, horticultural, turf, ornamental, home and garden, veterinary and forestry applications.

The silicone compositions of the present invention and its polymer can be used as a hydrolytically stable surfactant in pesticidal compositions at an amount sufficient to deliver between 0.005% and 2% to the final use concentration, either as a concentrate or diluted in a tank mix. Optionally the pesticidal composition may include excipients, cosurfactants, solvents, foam control agents, deposition aids, drift retardants, biologicals, micronutrients and fertilizers. The term pesticide means any compound used to destroy pests, e.g., rodenticides, insecticides, miticides, fungicides, and herbicides. Illustrative examples of pesticides that can be employed include, but are not limited to, growth regulators, photosynthesis inhibitors, pigment inhibitors, mitotic disrupters, lipid biosynthesis inhibitors, cell wall inhibitors, and cell membrane disrupters. The amount of pesticide employed in compositions of the invention varies with the type of pesticide employed. More specific examples of pesticide compounds that can be used with the compositions of the invention are, but not limited to, herbicides and growth regulators, such as: phenoxy acetic acids, phenoxy propionic acids, phenoxy butyric acids, benzoic acids, triazines and s-triazines, substituted ureas, uracils, bentazon, desmedipham, methazole, phenmedipham, pyridate, amitrole, clomazone, fluridone, norflurazone, dinitroanilines, isopropalin, oryzalin, pendimethalin, prodiamnine, trifluralin, glyphosate, sulfonylureas, imidazolinones, clethodim, diclofop-methyl, fenoxaprop-ethyl, fluazifop-p-butyl, haloxyfop-methyl, quizalofop, sethoxydim, dichlobenil, isoxaben, and bipyridylium compounds.

Fungicide compositions that can be used with the present invention include, but are not limited to, aldimorph, tridemorph, dodemorph, dimethomorph; flusilazol, azaconazole, cyproconazole, epoxiconazole, furconazole, propiconazole and tebuconazole; imazalil, thiophanate, benomyl carbendazim, chlorothialonil, dicloran, trifloxystrobin, fluoxystrobin, dimoxystrobin, azoxystrobin, furcaranil, prochloraz, flusulfamide, famoxadone, captan, maneb, mancozeb, dodicin, dodine, and metalaxyl.

Insecticide, larvacide, miticide and ovacide compounds that can be used with the composition of the present invention, but not limited to, Bacillus thuringiensis, spinosad, abamectin, doramectin, lepimectin, pyrethrins, carbaryl, primicarb, aldicarb, methomyl, amitraz, boric acid, chlordimeform, novaluron, bistrifluron, triflumuron, diflubenzuron, imidacloprid, diazinon, acephate, endosulfan, kelevan, dimethoate, azinphos-ethyl, azinphos-methyl, izoxathion, chlorpyrifos, clofentezine, lambda-cyhalothrin, permethrin, bifenthrin and cypermethrin.

Fertilizers and micronutrients include, but not limited to, zinc sulfate, ferrous sulfate, ammonium sulfate, urea, urea ammonium nitrogen, ammonium thiosulfate, potassium sulfate, monoammonium phosphate, urea phosphate, calcium nitrate, boric acid, potassium and sodium salts of boric acid, phosphoric acid, magnesium hydroxide, manganese carbonate, calcium polysulfide, copper sulfate, manganese sulfate, iron sulfate, calcium sulfate, sodium molybdate, calcium chloride,

The pesticide or fertilizer may be a liquid or a solid. If a solid, it is preferable that it is soluble in a solvent, or the organomodified siloxanes of the present invention, prior to application, and the silicone may act as a solvent, or surfactant for such solubility or additional surfactants may perform this function.

### III. Coatings:

Typically coatings formulations will require a wetting agent or surfactant for the purpose of emulsification, compatibilization of components, leveling, flow and reduction of surface defects. Additionally, these additives may provide improvements in the cured or dry film, such as improved abrasion resistance, antiblocking, hydrophilic, and hydrophobic properties. Coatings formulations may exist as, foul-resistant coatings, biological coatings, seed coatings, solvent-borne coatings, water-borne coatings and powder coatings.

The coatings components may be employed as: architecture coatings; OEM product coatings such as automotive coatings and coil coatings; Special Purpose coatings such as industrial maintenance coatings and marine coatings;

Typical resin types include: Polyesters, alkyds, acrylics, epoxies and combinations thereof.

### V. Personal Care:

The organomodified siloxane compositions of the present invention may be utilized in personal care emulsions, such as lotions, and creams. As is generally known, emulsions comprise at least two immiscible phases one of which is continuous and the other is discontinuous. Furthermore, emulsions may be liquids with varying viscosities or solids. Additionally, the particle size of the emulsions may render them microemulsions or miniemulsions and, when the particle sizes are sufficiently small, these emulsions may be transparent. Further, it is also possible to prepare emulsions of emulsions and these are generally known as multiple emulsions. These emulsions may be: 1) Aqueous emulsions, where the discontinuous phase comprises water and the continuous phase comprises the organomodified siloxane composition of the present invention. 2) Aqueous emulsions, where the discontinuous phase comprises the organomodified siloxane composition of the present invention and the continuous phase comprises water. 3) Non-aqueous emulsions, where the discontinuous phase comprises a non-aqueous hydroxylic solvent and the continuous phase comprises the organomodified siloxane composition of the present invention and 4) Non-aqueous emulsions, where the continuous phase comprises a non-aqueous hydroxylic organic solvent and the discontinuous phase comprises the organomodified siloxane composition of the present invention.

The monomer of the instant invention can be polymerized using emulsion polymerization and the copolymer of these monomer obtained with acrylic comonomers forms excellent films which can be used for various skin care and hair care applications (for eg, mascara, styling gels and sun protection films). Suitable personal care compositions are made by combining, in a manner known in the art, for example, by mixing one or more of the above components with the organomodified siloxane composition of the present invention. Suitable personal care compositions may be in the form of a single phase or in the form of an emulsion, including oil-in-water, water-in-oil and anhydrous emulsions where the silicone phase may be either the discontinuous phase or the continuous phase, as well as multiple emulsions, such as, for example, oil-in water-in-oil emulsions and water-in-oil-in water-emulsions.

### VI. Home Care:

The organomodified siloxane composition of the present invention and its polymers can be used as surfactants in home care applications, such as, laundry detergent and fabric softener, dishwashing liquids, wood and furniture polish, floor polish, tub and tile cleaners, toilet bowl cleaners, hard surface cleaners, window cleaners, antifog agents, drain cleaners, auto-dish washing detergents and sheeting agents, carpet cleaners, prewash spotters, rust cleaners and scale removers.

### SYNTHETIC EXAMPLES

The following examples are illustrative only and should not be construed as limiting the invention, which is properly delineated in the appended claims. Mono-methacrylated carbosiloxane monomers having different functionality were produced as shown below.

### MONOMER PREPARATION

### EXAMPLE 1:

### Synthesis of compound represented by the formula

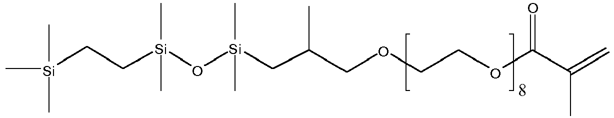

This monomer was prepared using two-step process. In the first step, a hydrosilylation reaction occurs between hydroxyl terminated methallyl polyether and mono-hydride functional carbosiloxane. In the second step, the hydroxyl group is converted into polymerizable methacrylate group through a methacrylation reaction. The mono-hydride functional carbosiloxane was prepared using the process disclosed in US 7,259,220 B1.

In a specific process, 1-(2-trimethylsilylethyl)-1,1,3,3-tetramethyldisiloxane (25 g), a hydride functional carbosiloxane, and a methallyl-terminated polyethylene glycol (46 g), having an average of 8 ethylene oxide (EO) units in the chain, were introduced into 250 mL three-neck round bottom (RB) flask equipped with a reflux condenser, mechanical stirrer, temperature controller with thermocouple and a nitrogen inlet. The contents were heated to 80°C to 90°C in presence of Karstedt's catalyst (50 to 100 ppm of Pt with respect to total reactant charge) and buffer (US 5,986,122) to prevent side reactions like dehydrocoupling reaction from taking place. After completion of the hydrosilylation, volatile compounds were removed from the reaction product under reduced pressure. The final product, hydroxyl terminated carbosiloxane polyether, was obtained as a colorless, transparent liquid in quantitative yield without any undesired side products. The resultant pure product was well characterized by multinuclear NMR spectroscopy. Synthesis of the silicone polyethers of the present invention can occur with or without solvent. If solvents are used, preferred ones include toluene, isopropyl alcohol or methyl ethyl ketone.
H-NMR (ppm): 0.02 (Si(CH₃), 0.3 & 0.6 (SiCH₂CH), 0.4 (SiCH₂CH₂Si), 1.0 (Si(CH₃), 1.9 (-CH<), 3.2 & 3.3 (>CH-CH₂-O-), 3.6 (-CH₂CH₂O-).
Si-NMR (ppm): 3.4 (Si(CH₃)₃CH₂), 7.2 (O-Si(CH₃)₂(CH₂)), 8.5 (O-Si(CH₃)₂(CH₂CH₂).

Next, the carbosiloxane polyether that was synthesized in the step above, triethylamine (11.3 g) and methylethylketone (100 ml) were introduced into three-neck one liter RB flask equipped with dropping funnel and a stirring blade. The flask was immersed in an ice bath and methacryloyl chloride (11.2 g) was added drop wise using dropping funnel over a period of approximately 1 hour with constant stirring. After completion of the addition the stirring was continued for another 3 hours at room temperature. The triethylamine hydrochloride salt that precipitated out during the reaction was filtered off. The solvent was removed with a rotary vacuum evaporator and the final monomer was obtained as colorless to pale yellow, transparent liquid. The low boiling point of the solvent used enables the solvent to be removed completely at a temperature of about 30°C to 40°C under vacuum (i.e. less than about 13333.22 Pa/10 mm Hg). The resulting monomer was well characterized by infrared spectroscopy, multinuclear NMR spectroscopy.
H-NMR (ppm): 0.02 (Si(CH₃), 0.3 & 0.6 (SiCH₂CH), 0.4 (SiCH₂CH₂Si), 0.98 (Si(CH₃), 1.98 (CH₃), 3.1 & 3.3 (>CH-CH₂-O-), 3.64 (-CH₂CH₂O-), 4.2 (CH₂COO), 5.6 & 6.15 (CH₂=).
Si-NMR (ppm): 3.5 (Si(CH₃)₃CH₂), 7.2 (O-Si(CH₃)₂(CH₂)), 8.4 (O-Si(CH₃)₂(CH₂CH₂).

### EXAMPLE 2:

### Synthesis of compound represented by the formula

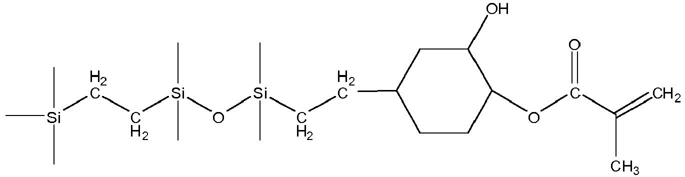

This monomer was prepared was also prepared using two-step process. In the first step, a hydrosilylation reaction occurs between hydride functional carbosiloxane and vinyl functional cyclohexene epoxide. In the second step, the epoxide group is reacted with unsaturated acids to introduce polymerizable group in it.

In a specific process, 1-(2-trimethylsilylethyl)-1,1,3,3-tetramethyldisiloxane (25 g) and vinyl cyclohexene epoxide (13.2 g) were introduced into 250 mL three-neck round bottom (RB) flask equipped with a reflux condenser, mechanical stirrer, temperature controller with thermocouple and a nitrogen inlet. The contents were heated to 80°C to 90°C in presence of Karstedt's catalyst (10 to 50 ppm Pt with respect to total reactant charge) and buffer (US 5,986,122) to prevent side reactions like dehydrocoupling reaction from taking place. After completion of the hydrosilylation, distilling out unwanted volatile compounds under reduced pressure purified the reaction product. The final product, epoxy functional carbosiloxane, was obtained as colorless, transparent liquid in quantitative yield without any undesired side products. The resultant pure product was well characterized by proton NMR spectroscopy. The epoxy functional carbosiloxane of the present invention can occur with or without solvent. If solvents are used, preferred ones include toluene, isopropylalcohol or methyl ethyl ketone.
H-NMR (ppm): 0.02 (Si(CH₃), 0.4 (SiCH₂CH₂Si), 0.5 (SiCH2), 1.2 to 2 (CH₂).

Next, the epoxy functional carbosiloxane synthesized above, titanium isopropoxide (0.4 wt% with respect to carbosiloxane) and hydroquinone (0.0025 wt% with respect to carbosiloxane) were introduced into three-neck one liter RB flask equipped with dropping funnel and a stirring blade. The flask was heated to 90 deg C in an oil bath and then acrylic acid (7.68 g) was added in a drop wise manner into the RB with constant stirring. After completion of the addition the stirring was continued for another 5 hours at 90 deg C. The solvent (toluene) and other volatile impurities were removed with a rotary vacuum evaporator and the final monomer was obtained as colorless, transparent liquid. The resulting mono-acrylated carbosiloxane monomer was well characterized by infrared spectroscopy, proton NMR spectroscopy.
H-NMR (ppm): 0.02 (Si(CH₃), 0.4 (SiCH₂CH₂Si), 0.5 (SiCH₂), 1.2 to 2 (CH₂), 3.8 & 4.8 (CH₂), 5.8, 6.2 & 6.4 (CH₂=CH-).

### EXAMPLE 3

### FORMATION OF SILICONE-HYDROGEL FILMS

The compound obtained in Example 1 (49 parts by weight), 2-hydroxy ethyl methacrylate (49 parts by weight), ethylene glycol dimethacrylate (EGDMA) (1 part by weight), and benzoyl peroxide (1 part by weight) were mixed and stirred. The resulting clear, homogeneous and transparent reaction mixture was purged with nitrogen gas and poured into a stainless steel mould. The thin film of the reaction mixture was thermally cured at 85°C for 8 hours using hot air oven. The silicone hydrogel film thus produced was transparent and water absorbing.

### EXAMPLE 4

A silicone-hydrogel film was obtained in the same way as in Example 3 except that the compound obtained in Example 2 was used instead of compound obtained in Example 1. The silicone hydrogel film thus produced was transparent and water absorbing.

### Comparative Example 1 (CEx. 1)

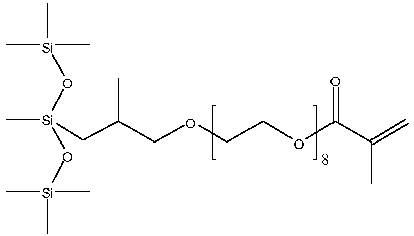

The monomer was prepared in the same way as in Example 1 except that 1,1,1,3,5,5,5-heptamethyltrisiloxane was used instead of 1-(2-trimethylsilylethyl)-1,1,3,3-tetramethyldisiloxane. The mono-acrylated siloxane monomer produced was well characterized by infrared spectroscopy, multinuclear NMR spectroscopy.
H-NMR (ppm): 0.02 (Si(CH₃), 0.3 & 0.6 (SiCH₂CH), 0.98 (Si(CH₃), 1.98 (CH₃), 3.1 & 3.3 (>CH-CH₂-O-), 3.64 (-CH₂CH₂O-), 4.3 (CH₂COO), 5.6 & 6.15 (CH₂=).
Si-NMR (ppm): 8 (-Si(CH₃)₃), -22 (O-Si(CH₃)₂-).

Hydrolytic stability of the monomer of the present invention was measured using HPLC (US Pub. Pat Appl. 20100069279). 0.5wt% of the monomers obtained in Example 1 (Ex.1) is introduced into three different vials containing 6.5, 7 and 7.5 pH solutions. The vials were sealed with leak proof seal and heated to 85 deg C. The heat accelerated hydrolytic degradation composition changes were monitored using HPLC as a function of time. The monomer of the current invention (Ex.1) showed improved hydrolytic stability under acidic, basic and neutral conditions in comparison to the conventional siloxane monomer (CEx.1) over the acidic, neutral and basic pH conditions.

Figure 1 show a hydrolytic stability data obtained for monomers produced in Ex.1 and CEx.2 at 85°C under different pH conditions; (A) pH 6.5, (B) pH 7.0 and (C) pH 7.5. As can be seen from the graphs in Figure 1, the monomer of the current invention (Ex.1) showed improved hydrolytic stability under acidic, basic and neutral conditions in comparison to the conventional siloxane monomer (CEx.2) over the acidic, neutral and basic pH conditions (Figure 1).

In the following the present invention will be described in preferred items:
1. A silicone composition comprising of a monomer having at least one carbosiloxane linkage having the general formulae (I):

   (R¹R²R³)Si-Y¹-[Si(R⁴R⁵)(Y²)]ₐ-Si(R⁶R⁷)- Z (I)

   wherein a is up to about 100;
   Y¹ is a substituted or unsubstituted divalent alkyl linking group of 1 to about 10 carbon atoms;
   Y² is oxygen;
   R¹, R², R³, R⁴, R⁵, R⁶, and R⁷ is independently selected from the group consisting of monovalent aliphatic, cycloaliphatic or aromatic or halogenated hydrocarbon groups of 1 to about 10 carbons;
   Z has the following general formulae (II)

      -R¹¹-B-X (II)

      wherein R¹¹ is a branched, divalent alkyl linking group having up to about 20 carbon atoms, B is a divalent hydrophilic functional moiety consisting of aliphatic, cycloaliphatic or aromatic hydrocarbons containing hetero atoms and X a polymerizable group selected from the group consisting of substituted or unsubstituted unsaturated aliphatic or aromatic hydrocarbons, acrylates and methacrylates, or
      wherein R¹¹ is a linear, divalent alkyl linking group having up to about 20 carbon atoms or a fluorinated hydrocarbon, an aralkyl or arylalkyl group, B is a divalent hydrophilic or hydrophobic functional moiety selected from the group of the following divalent moieties

      -O-(C₂H₄O)ₚ-(C₃H₆O)_{q}-(C₄H₈O)ᵣ-

      wherein p and q are independently 0 to about 100; r is 0 to about 50 and (p +q + r) is greater than 0, 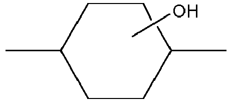 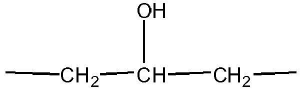 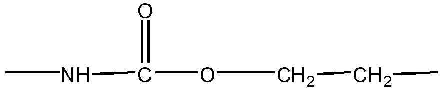 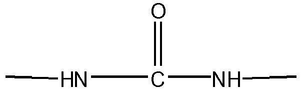 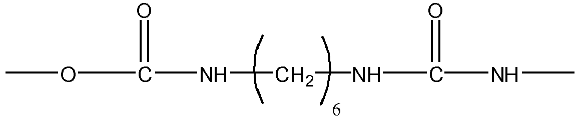 and X is a polymerizable group selected from the group consisting of substituted or unsubstituted unsaturated aliphatic or aromatic hydrocarbons, acrylates and methacrylates.
2. The silicone composition of item 1, wherein R¹, R², R³, R⁴, R⁵, R⁶, R⁷, independently comprises a saturated monovalent hydrocarbon group of 1 to about 9 carbon atoms, a fluorinated hydrocarbon, a aralkyl or arylalkyl group..
3. The silicone composition of item 1, wherein B comprises at least one functional group consisting of alkyl, alcohol, ether, ester, amide, amine, acid and its salts, cyano, thio, urethane, urea, carbonate, carbamate, sulfonate, sulphonamide, and phosphate.
4. The silicone composition of item 1, wherein B is one of the following divalent moieties

   -O-(C₂H₄O)ₚ-(C₃H₆O)_{q}-(C₄H₈O)ᵣ-

   Polyether
   wherein p and q are independently 0 to about 100; r is 0 to about 50 and (p +q + r) is greater than 0; 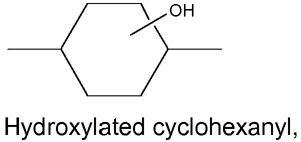 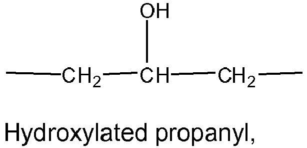 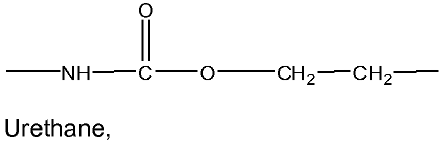 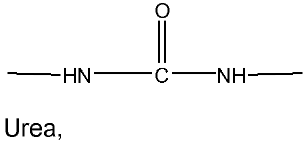 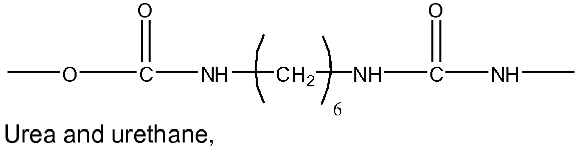 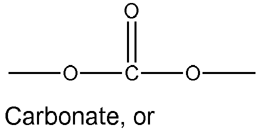 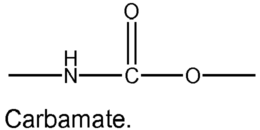
5. The silicone composition of item 1 wherein X is a polymerizable moiety having the general formula (III) 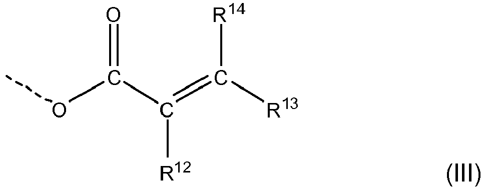 wherein R¹², R¹³, and R¹⁴ is hydrogen or a substituted or unsubstituted saturated monovalent hydrocarbon or halogenated hydrocarbon group of 1 to about 20 carbons.
6. The water-soluble and water-dispersible carbosiloxane compositions of item 1 comprising a hydrophilic polyethylene glycol and having the general formulae shown below: 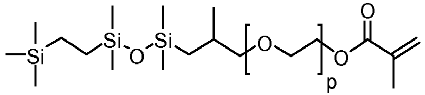 wherein p is greater than 5.
7. A carbosiloxane composition of item 1 with formula 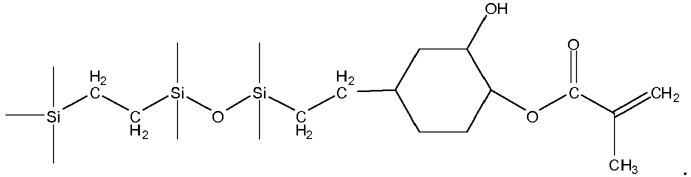
8. The silicone composition of item 1 wherein one of R¹-R⁷ comprises a fluorinated hydrocarbon.
9. The silicone composition of item 1 wherein B comprises at least one functional group consisting of alcohols, ethers, esters, amides, amines, acids and its salts, cyano, thio, urethane, urea, carbonate, carbamate, sulfonates, sulphonamides, and phosphates.
10. A health care composition comprising the silicone composition of Item 1 wherein the health care formulation comprises bioactives, anti-acne agents, anti-ageing agents, anti-caries agents, anti-fungal agents, anti-microbial agents, anti-oxidants, anti-cancer, anti-viral, anti-inflammatory, anti-coagulants, hemostatic agents, exfoliants, hormones, enzymes, medicinal compounds, biocides, external analgesics, oral care agents, oral care drugs, oxidizing agents, reducing agents, skin protectants, essential oils, insect repellents, UV light absorbing agents, solar filters, pigments, hydrating agents, vitamins and their combinations thereof.
11. A health care composition of item 10 comprising the silicone composition of item 1, can be used for health care application comprises hydrogels, drug delivery systems, transdermal patches, wound healing patches, wound dressing patches, puncta plugs, ophtha coils, retinal implants, transdermal iontophoresis, scaffold for tissue engineering, anti-microbial devices, anti-bacterial devices, biofilm resistant coatings, anti-fungal devices, wound management devices, ophthalmic devices, bioinserts, prostheses and body implants.
12. The personal care composition comprising the silicone composition of Item 1, wherein personal care formulation comprises surfactants, emulsifiers, solvents, emollients, moisturizers, humectants, pigments, colorants, fragrances, biocides, preservatives, chelating agents, antioxidants, anti-microbial agents, anti-fungal agents, antiperspirant agents, exfoliants, hormones, enzymes, medicinal compounds, vitamins, alpha-hydroxy acids, beta-hydroxy acids, retinols, niacinamide, skin lightening agents, salts, electrolytes, alcohols, polyols, absorbing agents for ultraviolet radiation, botanical extracts, organic oils, waxes, thickening agents, particulate fillers, silicones, clays, plasticizers, occlusives, sensory enhancers, esters, resins, film formers, film forming emulsifiers, high refractive index materials and their combinations thereof.
13. A personal care composition of item 12 comprising the silicone composition of item 1, which can be used for personal care application comprises antiperspirant/deodorants, including sprays, sticks and roll-on products, shaving products, skin lotions, moisturizers, toners, bath products, cleansing products, shampoos, conditioners, combined shampoo/conditioners, mousses, styling gels, hair sprays, hair dyes, hair color products, hair bleaches, waving products, hair straighteners, nail polish, nail polish remover, nail creams and lotions, cuticle softeners, sunscreen, insect repellent, anti-aging products, lipsticks, foundations, face powders, eye liners, eye shadows, blushes, makeup, mascaras, moisturizing preparations, foundations, body and hand preparations, skin care preparations, face and neck preparations, tonics, dressings, hair grooming aids, aerosol fixatives, fragrance preparations, aftershaves, make-up preparations, soft focus applications, night and day skin care preparations, non-coloring hair preparations, tanning preparations, synthetic and non-synthetic soap bars, hand liquids, nose strips, non-woven applications for personal care, baby lotions, baby baths and shampoos, baby conditioners, shaving preparations, cucumber slices, skin pads, make-up removers, facial cleansing products, cold creams, sunscreen products, mousses, spritzes, paste masks and muds, face masks, colognes and toilet waters, hair cuticle coats, shower gels, face and body washes, personal care rinse-off products, gels, foam baths, scrubbing cleansers, astringents, nail conditioners, eye shadow sticks, powders for face or eye, lip balms, lip glosses, hair care pump sprays and other non-aerosol sprays, hair-frizz-control gels, hair leave-in conditioners, hair pomades, hair de-tangling products, hair fixatives, hair bleach products, skin lotions, pre-shaves and pre-electric shaves, anhydrous creams and lotions, oil/water, water/oil, multiple and macro and micro emulsions, water-resistant creams and lotions, anti-acne preparations, mouth-washes, massage oils, toothpastes, clear gels and sticks, ointment bases, topical wound-healing products, aerosol talcs, barrier sprays, vitamin and anti-aging preparations, herbal-extract preparations, bath salts, bath and body milks, hair styling aids, hair-, eye-, nail-and skin-soft solid applications, controlled-release personal care products, hair conditioning mists, skin care moisturizing mists, skin wipes, pore skin wipes, pore cleaners, blemish reducers, skin exfoliators, skin desquamation enhancers, skin towelettes and cloths, depilatory preparations, personal care lubricants, nail coloring preparations, sunscreens, cosmetics, hair care products, skin care products, toothpastes, drug delivery systems for topical application of medicinal compositions that are to be applied to the skin and combinations comprises at least one of the foregoing applications.
14. A pesticide formulation comprises the silicone composition of item 1.
15. A fertilizer formulation comprises the silicone composition of item 1.
16. A fertilizer coating, seed coating, anti-fouling coating, waterborne coating formulation comprising of the silicone composition of item 1.
17. A process of making the silicone composition of item 1 comprising reacting a silicone-containing compound having the general formulae (IV):

   (R¹R²R³)Si-Y¹-[Si(R⁴R⁵)(Y²)]ₐ-Si(R⁶R⁷)-H (IV)

   wherein a is 0 to 100;
   Y¹ is a substituted or unsubstituted divalent alkyl linking group of 1 to 10 carbon atoms;
   Y² is oxygen;
   R¹, R², R³, R⁴, R⁵, R⁶, and R⁷ is independently selected from the group consisting of monovalent aliphatic, cycloaliphatic or aromatic or halogenated hydrocarbon groups of 1 to about 10 carbons;
   with terminally unsaturated group having the general formulae (V):

      R¹⁵-B-M (V)

      wherein R¹⁵ is branched unsaturated alkyl groups having up to about 20 carbon atoms, B is a divalent hydrophilic functional moiety consisting of aliphatic, cycloaliphatic or aromatic hydrocarbons containing hetero atoms,
      wherein B comprises functionalities selected from the group consisting of alkyl, alcohol, ether, ester, amide, amine, acid and its salts, cyano, this, urethane, urea, carbonate, carbamate, sulfonate, sulphonamide, phosphate and combinations thereof;
      M is selected from the group consisting of hydroxyl, halogen, epoxy and carboxylic acid group to produce functionalized carbosiloxane;
      and reacting the functionalized carbosiloxane with an alkylacryloyl compound having the following formula (VI): 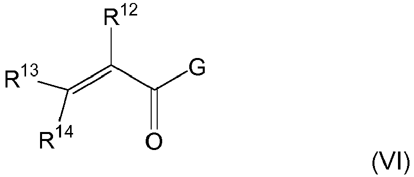 wherein G is selected from the group consisting of a halogen, hydroxyl and alkyloxy having 1 to about 10 carbon atoms and R¹², R¹³ and R¹⁴ is independently selected from the group consisting of hydrogen, substituted saturated monovalent hydrocarbons having 1 to about 20 carbons and unsubstituted saturated monovalent hydrocarbons having 1 to about 20 carbons to produce said silicone monomer having the general formulae (I) of item 1.
18. The process of item 17, wherein said reaction of said functionalized carbosiloxane with said alkylacryloyl compound is carried out in the presence of at least one tertiary base or at least one ionic exchange resin (IER) and a low boiling polar solvent. The process of Item 17 wherein the amount of carbosiloxane reacted is present in an amount that ranges from about 5 to about 90 weight percent of the total composition, and the amount of alkyacryloyl is present in an amount that ranges from about 5 to about 80 weight percent of the total composition.
19. A health care composition comprising the silicone composition prepared from the process of item 17, wherein the health care formulation comprises bioactives, anti-acne agents, anti-ageing agents, anti-caries agents, anti-fungal agents, anti-microbial agents, anti-oxidants, anti-cancer, anti-viral, anti-inflammatory, anti-coagulants, hemostatic agents, exfoliants, hormones, enzymes, medicinal compounds, biocides, external analgesics, oral care agents, oral care drugs, oxidizing agents, reducing agents, skin protectants, essential oils, insect repellents, UV light absorbing agents, solar filters, pigments, hydrating agents, vitamins and their combinations thereof.
20. A health care composition of item 19 comprising the silicone composition prepared from the process of item 17, can be used for health care application comprises hydrogels, drug delivery systems, transdermal patches, wound healing patches, wound dressing patches, puncta plugs, ophtha coils, retinal implants, transdermal iontophoresis, scaffold for tissue engineering, anti-microbial devices, anti-bacterial devices, biofilm resistant coatings, anti-fungal devices, wound management devices, ophthalmic devices, bioinserts, prostheses and body implants.
21. The personal care composition comprising the silicone composition prepared from the process of item 17, wherein personal care formulation comprises surfactants, emulsifiers, solvents, emollients, moisturizers, humectants, pigments, colorants, fragrances, biocides, preservatives, chelating agents, antioxidants, anti-microbial agents, anti-fungal agents, antiperspirant agents, exfoliants, hormones, enzymes, medicinal compounds, vitamins, alpha-hydroxy acids, beta-hydroxy acids, retinols, niacinamide, skin lightening agents, salts, electrolytes, alcohols, polyols, absorbing agents for ultraviolet radiation, botanical extracts, organic oils, waxes, thickening agents, particulate fillers, silicones, clays, plasticizers, occlusives, sensory enhancers, esters, resins, film formers, film forming emulsifiers, high refractive index materials and their combinations thereof.
22. A personal care composition of item 21 comprising the silicone composition prepared from the process of item 17, can be used for personal care application comprises antiperspirant/deodorants, including sprays, sticks and roll-on products, shaving products, skin lotions, moisturizers, toners, bath products, cleansing products, shampoos, conditioners, combined shampoo/conditioners, mousses, styling gels, hair sprays, hair dyes, hair color products, hair bleaches, waving products, hair straighteners, nail polish, nail polish remover, nail creams and lotions, cuticle softeners, sunscreen, insect repellent, anti-aging products, lipsticks, foundations, face powders, eye liners, eye shadows, blushes, makeup, mascaras, moisturizing preparations, foundations, body and hand preparations, skin care preparations, face and neck preparations, tonics, dressings, hair grooming aids, aerosol fixatives, fragrance preparations, aftershaves, make-up preparations, soft focus applications, night and day skin care preparations, non-coloring hair preparations, tanning preparations, synthetic and non-synthetic soap bars, hand liquids, nose strips, non-woven applications for personal care, baby lotions, baby baths and shampoos, baby conditioners, shaving preparations, cucumber slices, skin pads, make-up removers, facial cleansing products, cold creams, sunscreen products, mousses, spritzes, paste masks and muds, face masks, colognes and toilet waters, hair cuticle coats, shower gels, face and body washes, personal care rinse-off products, gels, foam baths, scrubbing cleansers, astringents, nail conditioners, eye shadow sticks, powders for face or eye, lip balms, lip glosses, hair care pump sprays and other non-aerosol sprays, hair-frizz-control gels, hair leave-in conditioners, hair pomades, hair de-tangling products, hair fixatives, hair bleach products, skin lotions, pre-shaves and pre-electric shaves, anhydrous creams and lotions, oil/water, water/oil, multiple and macro and micro emulsions, water-resistant creams and lotions, anti-acne preparations, mouth-washes, massage oils, toothpastes, clear gels and sticks, ointment bases, topical wound-healing products, aerosol talcs, barrier sprays, vitamin and anti-aging preparations, herbal-extract preparations, bath salts, bath and body milks, hair styling aids, hair-, eye-, nail-and skin-soft solid applications, controlled-release personal care products, hair conditioning mists, skin care moisturizing mists, skin wipes, pore skin wipes, pore cleaners, blemish reducers, skin exfoliators, skin desquamation enhancers, skin towelettes and cloths, depilatory preparations, personal care lubricants, nail coloring preparations, sunscreens, cosmetics, hair care products, skin care products, toothpastes, drug delivery systems for topical application of medicinal compositions that are to be applied to the skin and combinations comprises at least one of the foregoing applications.
23. A pesticide formulation comprises the silicone composition prepared from the process of item 17.
24. A fertilizer formulation comprises the silicone composition prepared from the process of item 17.
25. A fertilizer coating, seed coating, anti-fouling coating, waterborne coating formulation comprising the silicone composition prepared from the process of item 17.

## Claims

1. A silicone composition comprising of a monomer having at least one carbosiloxane linkage having the general formulae (1):
(R¹R²R³)Si-Y¹-[Si(R⁴R⁵)(Y²)]ₐ-Si(R⁶R⁷)-Z (1)
wherein a is up to about 100;
Y¹ is a substituted or unsubstituted divalent alkyl linking group of 1 to about 10 carbon atoms;
Y² is oxygen;
R¹, R², R³, R⁴, R⁵, R⁶, and R⁷ is independently selected from the group consisting of monovalent aliphatic, cycloaliphatic or aromatic or halogenated hydrocarbon groups of 1 to about 10 carbons;
Z has the following general formulae (II)
-R¹¹-B-X (II)
wherein R¹¹ is a branched, divalent alkyl linking group having up to about 20 carbon atoms or a fluorinated hydrocarbon, a aralkyl or arylalkyl group, and B is a divalent hydrophilic functional moiety consisting of aliphatic, cycloaliphatic or aromatic hydrocarbons containing hetero atoms and
X is a polymerizable group selected from the group consisting of substituted or unsubstituted unsaturated aliphatic or aromatic hydrocarbons, acrylates and methacrylates, or wherein R¹¹ is a linear, divalent alkyl linking group having up to about 20 carbon atoms or a fluorinated hydrocarbon, an aralkyl or arylalkyl group, B is a divalent hydrophilic or hydrophobic functional moiety selected from the group of the following divalent moieties
-O-(C₂H₄O)ₚ-(C₃H₆O)_{q}-(C₄H₈O)ᵣ-
wherein p and q are independently 0 to about 100; r is 0 to about 50 and (p +q + r) is greater than 0, 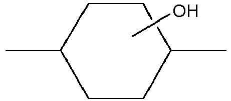 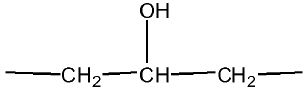 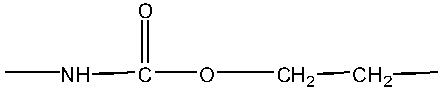 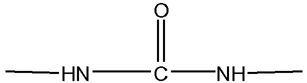 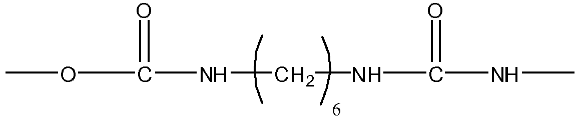 and X is a polymerizable group selected from the group consisting of substituted or unsubstituted unsaturated aliphatic or aromatic hydrocarbons, acrylates and methacrylates.

2. The silicone composition of claim 1, wherein each of R¹, R², R³, R⁴, R⁵, R⁶, R⁷, independently comprises a saturated monovalent hydrocarbon group of 1 to about 9 carbon atoms, a fluorinated hydrocarbon, an aralkyl or arylalkyl group, and R¹¹ independently comprises a saturated divalent hydrocarbon group of up to about 9 carbon atoms.

3. The silicone composition of claim 1, wherein B is selected from the group consisting of one of the following divalent moieties: -O-(C₂H₄O)ₚ-(C₃H₆O)_{q}-(C₄H₈O)ᵣ -wherein p and q are independently 0 to about 100; r is 0 to about 50 and (p +q + r) is greater than 0, 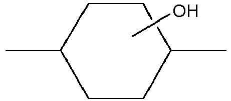 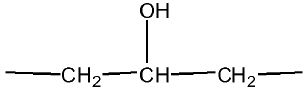 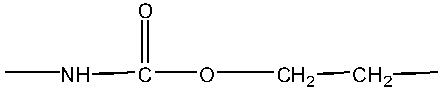 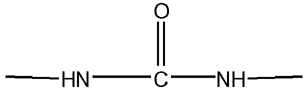 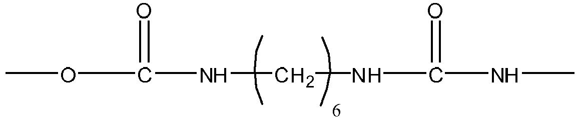 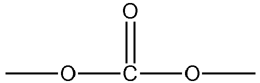 and

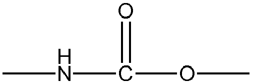

4. The silicone composition of claim 1 wherein X is a polymerizable moiety having the general formula (III) 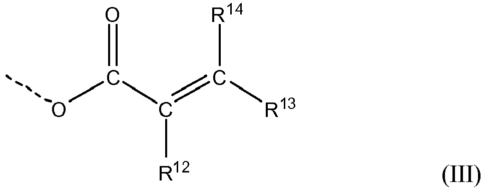 wherein R¹², R¹³, and R¹⁴ is hydrogen or a substituted or unsubstituted saturated monovalent hydrocarbon or halogenated hydrocarbon group of 1 to about 20 carbons.

5. The composition of claim 1 comprising a hydrophilic polyethylene glycol and having the general formulae shown below: 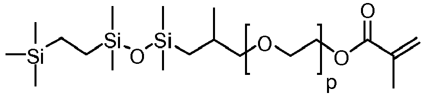 wherein p is greater than 5.

6. A composition of claim 1 with formula

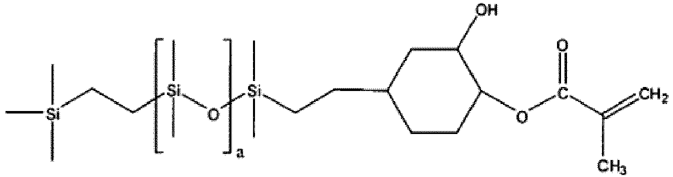

7. The silicone composition of claim 1 wherein one of R¹-R¹⁴ comprises a fluorinated hydrocarbon or
wherein each of R¹-R¹⁴ independently comprises at least one moiety selected from the group consisting of saturated monovalent aliphatic or aromatic hydrocarbon having from 1 to about 9 carbon atoms, or
wherein B comprises at least one functional group consisting of alcohols, ethers, esters, amides, amines, acids and its salts, cyano, thio, urethane, urea, carbonate, carbamate, sulfonates, sulphonamides and phosphates.

8. A process of making the monomer having the formula (I) of claim 1 comprising reacting a silicone-containing compound having the general formulae (IV):
(R¹R²R³)Si-Y¹-[Si(R⁴R⁵)(Y²)]ₐ-Si(R⁶R⁷)-H (IV)
wherein a is up to 100;
Y¹ is a substituted or unsubstituted divalent alkyl linking group of 1 to 10 carbon atoms;
Y² is oxygen;
R¹, R², R³, R⁴, R⁵, R⁶, and R⁷ is independently selected from the group consisting of monovalent aliphatic, cycloaliphatic or aromatic or halogenated hydrocarbon groups of 1 to about 10 carbons;
with terminally unsaturated group having the general formulae (V):
R¹⁵-B-M (V)
wherein R¹⁵ is branched unsaturated alkyl groups having up to about 20 carbon atoms, B is a divalent hydrophilic functional moiety consisting of aliphatic, cycloaliphatic or aromatic hydrocarbons and substituted or unsubstituted, saturated and unsaturated aliphatic, cycloaliphatic or aromatic hydrocarbons containing hetero atoms,
wherein B comprise functionalities selected from the group consisting of alkyl, alcohol, ether, ester, amide, amine, acid and its salts, cyano, thio, urethane, urea, carbonate, carbamate, sulfonate, sulphonamide, phosphate and combinations thereof;
M is selected from the group consisting of hydroxyl, halogen, epoxy and carboxylic acid group to produce functionalized carbosiloxane;
and reacting the functionalized carbosiloxane with an alkylacryloyl compound having the following formula (VI): 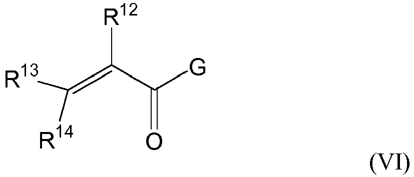 wherein G is selected from the group consisting of a halogen, hydroxyl and alkyloxy having 1 to about 10 carbon atoms and R¹², R¹³ and R¹⁴ is independently selected from the group consisting of hydrogen, substituted saturated monovalent hydrocarbons having 1 to about 20 carbons and unsubstituted saturated monovalent hydrocarbons having 1 to about 20 carbons to produce said silicone monomer having the general formulae (I) of claim 1.

9. The process of claim 8, wherein said reaction of said functionalized carbosiloxane in an amount that ranges from about 5 to about 90 weight percent of the total composition with said alkylacryloyl compound in an amount that ranges from about 5 to about 80 weight percent of the total composition is carried out in the presence of at least one tertiary base or at least one ionic exchange resin (IER) and a low boiling polar solvent.

10. A health care composition comprising the silicone composition of claim 1 or the monomer obtainable by the process of claim 8, which further comprises bioactives, anti-acne agents, anti-ageing agents, anti-caries agents, anti-fungal agents, anti-microbial agents, anti-oxidants, anti-cancer, anti-viral, anti-inflammatory, anti-coagulants, hemostatic agents, exfoliants, hormones, enzymes, medicinal compounds, biocides, external analgesics, oral care agents, oral care drugs, oxidizing agents, reducing agents, skin protectants, essential oils, insect repellents, UV light absorbing agents, solar filters, pigments, hydrating agents, vitamins and their combinations thereof.

11. A health care product comprising the silicone composition of claim 1 or the monomer obtainable by the process of claim 8 wherein hydrogels, drug delivery systems, transdermal patches, wound healing patches, wound dressing patches, puncta plugs, ophtha coils, retinal implants, transdermal iontophoresis, scaffold for tissue engineering, anti-microbial devices, anti-bacterial devices, biofilm resistant coatings, anti-fungal devices, wound management devices, ophthalmic devices, bioinserts, prostheses and body implants are comprised.

12. The personal care composition comprising the silicone composition of claim 1 or the monomer obtainable by the process of claim 8, which further comprises surfactants, emulsifiers, solvents, emollients, moisturizers, humectants, pigments, colorants, fragrances, biocides, preservatives, chelating agents, antioxidants, anti-microbial agents, anti-fungal agents, antiperspirant agents, exfoliants, hormones, enzymes, medicinal compounds, vitamins, alpha-hydroxy acids, beta-hydroxy acids, retinols, niacinamide, skin lightening agents, salts, electrolytes, alcohols, polyols, absorbing agents for ultraviolet radiation, botanical extracts, organic oils, waxes, thickening agents, particulate fillers, silicones, clays, plasticizers, occlusives, sensory enhancers, esters, resins, film formers, film forming emulsifiers, high refractive index materials and their combinations thereof.

13. A personal care product comprising the silicone composition of claim 1 or the monomer obtainable by the process of claim 8 wherein antiperspirant/deodorants, including sprays, sticks and roll-on products, shaving products, skin lotions, moisturizers, toners, bath products, cleansing products, shampoos, conditioners, combined shampoo/conditioners, mousses, styling gels, hair sprays, hair dyes, hair color products, hair bleaches, waving products, hair straighteners, nail polish, nail polish remover, nail creams and lotions, cuticle softeners, sunscreen, insect repellent, anti-aging products, lipsticks, foundations, face powders, eye liners, eye shadows, blushes, makeup, mascaras, moisturizing preparations, foundations, body and hand preparations, skin care preparations, face and neck preparations, tonics, dressings, hair grooming aids, aerosol fixatives, fragrance preparations, aftershaves, make-up preparations, soft focus applications, night and day skin care preparations, non-coloring hair preparations, tanning preparations, synthetic and non-synthetic soap bars, hand liquids, nose strips, non-woven applications for personal care, baby lotions, baby baths and shampoos, baby conditioners, shaving preparations, cucumber slices, skin pads, make-up removers, facial cleansing products, cold creams, sunscreen products, mousses, spritzes, paste masks and muds, face masks, colognes and toilet waters, hair cuticle coats, shower gels, face and body washes, personal care rinse-off products, gels, foam baths, scrubbing cleansers, astringents, nail conditioners, eye shadow sticks, powders for face or eye, lip balms, lip glosses, hair care pump sprays and other non-aerosol sprays, hair-frizz-control gels, hair leave-in conditioners, hair pomades, hair de-tangling products, hair fixatives, hair bleach products, skin lotions, pre-shaves and pre-electric shaves, anhydrous creams and lotions, oil/water, water/oil, multiple and macro and micro emulsions, water-resistant creams and lotions, anti-acne preparations, mouth-washes, massage oils, toothpastes, clear gels and sticks, ointment bases, topical wound-healing products, aerosol talcs, barrier sprays, vitamin and anti-aging preparations, herbal-extract preparations, bath salts, bath and body milks, hair styling aids, hair-, eye-, nail-and skin-soft solid applications, controlled-release personal care products, hair conditioning mists, skin care moisturizing mists, skin wipes, pore skin wipes, pore cleaners, blemish reducers, skin exfoliators, skin desquamation enhancers, skin towelettes and cloths, depilatory preparations, personal care lubricants, nail coloring preparations, sunscreens, cosmetics, hair care products, skin care products, toothpastes, drug delivery systems for topical application of medicinal compositions that are to be applied to the skin are comprised.

14. A pesticide formulation, a fertilizer formulation, a fertilizer coating, seed coating, anti-fouling coating or waterborne coating formulation comprising the silicone composition of claim 1 or the monomer obtainable by the process of claim 8.

## Patentansprüche

1. Eine Silikonzusammensetzung, umfassend ein Monomer, das über mindestens eine Carbosiloxan-Verknüpfung verfügt, das die allgemeine Formel (I) aufweist:
(R¹R²R³)Si-Y¹-[Si(R⁴R⁵)(Y²)]ₐ-Si(R⁶R⁷)-Z (I)
worin a bis zu etwa 100 ist;
Y¹ eine substituierte oder unsubstituierte zweiwertige Alkyl-Verknüpfungsgruppe mit 1 bis 10 Kohlenstoffatomen ist;
Y² Sauerstoff ist;
R¹, R², R³, R⁴, R⁵, R⁶ und R⁷ in unabhängiger Weise aus der Gruppe, die aus einwertigen aliphatischen, cycloaliphatischen oder aromatischen oder halogenierten Kohlenwasserstoffgruppen mit 1 bis 10 Kohlenstoffatomen besteht, ausgewählt sind;
Z die folgende allgemeine Formel (II)
-R¹¹-B-X (II)
hat,
worin R¹¹ eine verzweigte, zweiwertige Alkyl-Verknüpfungsgruppe, die bis zu etwa 20 Kohlenstoffatome aufweist, oder ein fluorierter Kohlenwasserstoff, eine Aralkyl- oder Arylalkylgruppe ist, und B eine zweiwertige hydrophile funktionelle Gruppe ist, die aus aliphatischen, cycloaliphatischen oder aromatischen Kohlenwasserstoffen, die Heteroatome enthalten, besteht, und
X eine polymerisierbare Gruppe ist, die aus der Gruppe ausgewählt ist, die aus substituierten oder unsubstituierten ungesättigten aliphatischen oder aromatischen Kohlenwasserstoffen, Acrylaten und Methacrylaten besteht,
oder worin R¹¹ eine lineare, zweiwertige Alkyl-Verknüpfungsgruppe, die bis zu etwa 20 Kohlenstoffatome aufweist, oder ein fluorierter Kohlenwasserstoff, eine Aralkyl- oder Arylalkylgruppe ist, B eine zweiwertige hydrophile oder hydrophobe funktionelle Gruppe ist, die aus der Gruppe der folgenden zweiwertigen Gruppen
-O-(C₂H₄O)ₚ-(C₃H₆O)_{q}-(C₄H₈O)ᵣ-
worin p und q in unabhängiger Weise 0 bis etwa 100 sind; r 0 bis etwa 50 ist und (p + q + r) größer ist als 0, 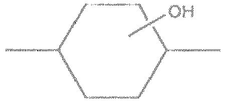 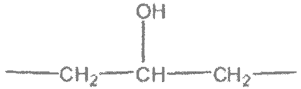 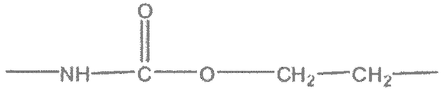 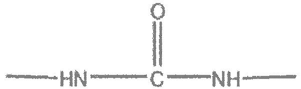 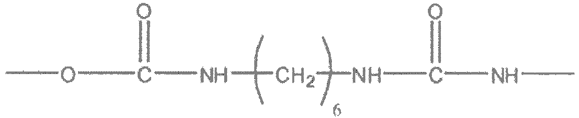 ausgewählt ist,
und X eine polymerisierbare Gruppe ist, die aus der Gruppe ausgewählt ist, die aus subtiuierten oder unsubstituierten ungesättigten aliphatischen oder aromatischen Kohlenwasserstoffen, Acrylaten und Methacrylaten besteht.

2. Die Silikonzusammensetzung gemäß Anspruch 1, worin R¹, R², R³, R⁴, R⁵, R⁶ und R⁷ jeweils in unabhängiger Weise eine gesättigte einwertige Kohlenwasserstoffgruppe von 1 bis ungefähr 9 Kohlenstoffatomen, einen fluorierten Kohlenwasserstoff, eine Aralkyl- oder Arylalkylgruppe umfasst, und R¹¹ in unabhängiger Weise eine gesättigte zweiwertige Kohlenwasserstoffgruppe von bis zu ungefähr 9 Kohlenstoffatomen umfasst.

3. Die Silikonzusammensetzung gemäß Anspruch 1, worin B aus der Gruppe ausgewählt ist, die aus einer der folgenden zweiwertigen Gruppen besteht:
-O-(C₂H₄O)ₚ-(C₃H₆O)_{q}-(C₄H₈O)ᵣ-
worin p und q jeweils in unabhängiger Weise 0 bis ungefähr 100 sind; r 0 bis ungefähr 50 und (p + q + r) größer als 0 ist, 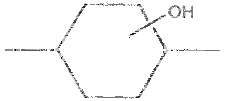 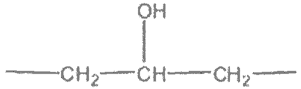 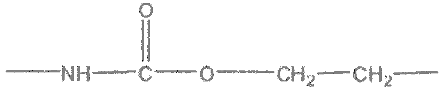 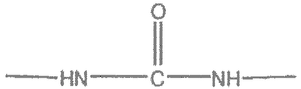 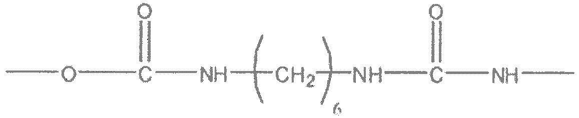 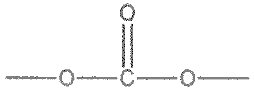 und

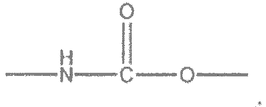

4. Die Silikonzusammensetzung gemäß Anspruch 1, worin X eine polymerisierbare Gruppe ist, die die allgemeine Formel (III) 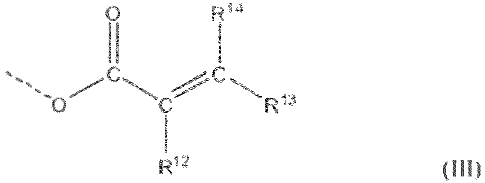 aufweist, worin es sich bei R¹², R¹³ und R¹⁴ um Wasserstoff oder eine substituierte oder unsubstituierte gesättigte einwertige Kohlenwasserstoffgruppe oder halogenierte Kohlenwasserstoffgruppe mit 1 bis etwa 20 Kohlenstoffatomen handelt.

5. Die Zusammensetzung gemäß Anspruch 1, die ein hydrophiles Polyethylenglycol umfasst und die unten dargestellte allgemeine Formel aufweist: 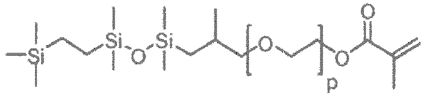 worin p größer als 5 ist.

6. Eine Zusammensetzung gemäß Anspruch 1 mit der Formel

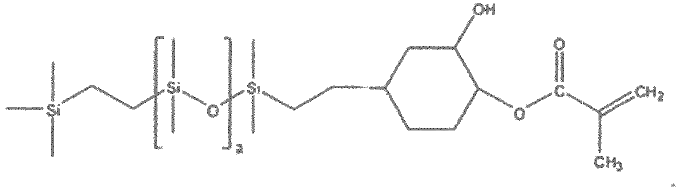

7. Die Silikonzusammensetzung gemäß Anspruch 1, worin eines von R¹-R¹⁴ einen fluorierten Kohlenwasserstoff umfasst, oder
worin R¹-R¹⁴ jeweils in unabhängiger Weise mindestens eine Gruppe umfasst, die aus der Gruppe auswählt ist, die aus einem gesättigten einwertigen alipatischen oder aromatischen Kohlenwasserstoff, der von 1 bis etwa 9 Kohlenstoffatome aufweist, besteht, oder
worin B mindestens eine funktionelle Gruppe umfasst, die aus Alkoholen, Ethern, Estern, Amiden, Aminen, Säuren und deren Salzen, Cyano, Thio, Urethan, Harnstoff, Carbonat, Carbamat, Sulfonaten, Sulfonamiden und Phosphaten besteht.

8. Ein Verfahren zur Herstellung des Monomers, das die Formel (I) gemäß Anspruch 1 aufweist, das das zur Reaktion Bringen einer Silikon-haltigen Verbindung, die die allgemeine Formel (IV) aufweist:
(R¹R²R³)Si-Y¹-(Si(R⁴R⁵)(Y²)]ₐ-Si(R⁶R⁷)-H (IV)
worin a bis zu 100 ist;
Y¹ eine substituierte oder unsubstituierte zweiwertige Alkyl-Verknüpfungsgruppe mit 1 bis 10 Kohlenstoffatomen ist;
Y² Sauerstoff ist;
R¹, R², R³, R⁴, R⁵, R⁶ und R⁷ in unabhängiger Weise aus der Gruppe, die aus einwertigen aliphatischen, cycloaliphatischen oder aromatischen oder halogenierten Kohlenwasserstoffgruppen mit 1 bis 10 Kohlenstoffatomen besteht, ausgewählt sind;
mit einer terminal ungesättigten Gruppe, die die allgemeine Formel (V) aufweist:
R¹⁵-B-M (V)
worin R¹⁵ eine verzweigte ungesättigte Alkylgruppe mit bis zu etwa 20 Kohlenstoffatomen ist, B eine zweiwertige hydrophile funktionelle Gruppe bestehend aus aliphatischen, cycloaliphatischen oder aromatischen Kohlenwasserstoffen und substituierten oder unsubstituierten, gesättigten oder ungesättigten aliphatischen, cycloaliphatischen oder aromatischen Kohlenwasserstoffen, die Heteroatome enthalten, ist,
wobei B funktionelle Gruppen umfasst, die aus der Gruppe, die aus Alkyl, Alkohol, Ether, Ester, Amid, Amin, Säure und deren Salze, Cyano, Thio, Urethan, Harnstoff, Carbonat, Carbamat, Sulfonat, Sulfonamid, Phosphat und Kombinationen davon besteht, ausgewählt sind;
M aus der Gruppe ausgewählt ist, die aus Hydroxyl-, Halogen-, Epoxy- und der Carbonsäuregruppe besteht,
um funktionalisierte Carbosiloxane herzustellen;
und das zur Reaktion Bringen der funktionalisierten Carbosiloxane mit einer Alkylacryloylverbindung, die die folgende Formel (VI) aufweist: 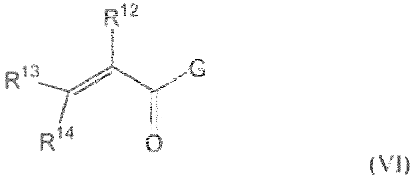 worin G aus der Gruppe ausgewählt ist, die aus Halogen, Hydroxyl und Alkyloxy, das 1 bis etwa 10 Kohlenstoffatome aufweist, ausgewählt ist, und R¹², R¹³ und R¹⁴ in unabhängiger Weise aus der Gruppe ausgewählt sind, die aus Wasserstoff, substituierten gesättigten einwertigen Kohlenwasserstoffen, die 1 bis etwa 20 Kohlenstoffatome aufweisen, und unsubstituierten gesättigten einwertigen Kohlenwasserstoffen, die 1 bis etwa 20 Kohlenstoffatome aufweisen, besteht, um die genannten Silikonmonomere, die die allgemeine Formel (I) gemäß Anspruch 1 aufweisen, herzustellen, umfasst.

9. Das Verfahren gemäß Anspruch 8, wobei die genannte Reaktion der genannten funktionalisierten Carbosiloxane in einer Menge, die von etwa 5 bis etwa 90 Gewichts-Prozent der Gesamtzusammensetzung reicht, mit der genannten Alkylacryloylverbindung in einer Menge, die von etwa 5 bis etwa 80 Gewichts-Prozent der Gesamtzusammensetzung reicht, in der Gegenwart mindestens einer tertiären Base und mindestens eines lonentauscher-Harzes (IER) und einem niedrig-siedenden polaren Lösungsmittel durchgeführt wird.

10. Eine Gesundheitspflegezusammensetzung, die die Silikonzusammensetzung gemäß Anspruch 1 oder das durch das Verfahren gemäß Anspruch 8 erhältliche Monomer umfasst, die darüber hinaus bioaktive Substanzen, Anti-Akne-Wirkstoffe, Anti-Aging-Wirkstoffe, Anti-Karies-Wirkstoffe, antimykotische Wirkstoffe, antimikrobielle Wirkstoffe, Anti-Oxidantien, Anti-Krebs-Mittel, antivirale Wirkstoffe, antiinflammatorische Wirkstoffe, gerinnungshemmende Mittel, blutstillende Mittel, Körperpeelings, Hormone, Enzyme, medizinische Verbindungen, Biozide, äußerlich anzuwendende Analgetika, Mundpflegemittel, Mundpflegemedikamente, Oxidationsmittel, Reduktionsmittel, Hautschutzmittel, ätherische Öle, Insektenschutzmittel, UV-Licht absorbierende Mittel, Sonnenfilter, Pigmente, hydratisierende Mittel, Vitamine und deren Kombinationen umfasst.

11. Ein Gesundheitspflegeprodukt, das die Silikonzusammensetzung gemäß Anspruch 1 oder das durch das Verfahren gemäß Anspruch 8 erhältliche Monomer umfasst, wovon Hydrogele, Systeme zur Abgabe von Arzneimitteln, Transdermalpflaster, Wundheilungspflaster, Wundauflagen, Tränenpunktstecker, Ophtha-Spiralen, Netzhautimplantate, transdermale lontophorese, ein Gerüst für Tissue Engineering, antimikrobielle Vorrichtungen, antibakterielle Vorrichtungen, Biofilm-beständige Beschichtungen, antimykotische Vorrichtungen, Wundversorgungsvorrichtungen, ophthalmologische Vorrichtungen, Bio-Inserts, Prothesen und Körperimplantate umfasst werden.

12. Die Körperpflegezusammensetzung, die die Silikonzusammensetzung gemäß Anspruch 1 oder das durch das Verfahren gemäß Anspruch 8 erhältliche Monomer umfasst, die darüber hinaus Tenside, Emulgatoren, Lösungsmittel, Weichmacher, Feuchtigkeitsspender, Feuchthaltemittel, Pigmente, Färbemittel, Duftstoffe, Biozide, Konservierungsstoffe, Komplexbildner, Antioxidantien, antimikrobielle Mittel, Antimykotika, Antitranspirant-Mittel, Körperpeelings, Hormone, Enzyme, medizinische Verbindungen, Vitamine, alpha-Hydroxysäuren, beta-Hydroxysäuren, Retinole, Niacinamid, Hautaufhellungsmittel, Salze, Elektrolyte, Alkohole, Polyole, Absorbtionsmittel für ultraviolette Strahlung, pflanzliche Extrakte, organische Öle, Wachse, partikelförmige Füllstoffe, Silikone, Tonerden, Weichmacher, Okklusivstoffe, sensorische Verstärker, Ester, Harze, Filmbildner, filmbildende Emulgatoren, Materialien mit hohem Refraktionsindex und Kombinationen derselben umfasst.

13. Ein Körperpflegeprodukt, das die Silikonzusammensetzung gemäß Anspruch 1 oder das durch das Verfahren gemäß Anspruch 8 erhältliche Monomer umfasst, wovon Antiperspirante/Deodorants inklusive Sprays, Sticks und Roll-on-Produkte, Rasurprodukte, Hautlotionen, Feuchtigkeitscremes, Toner, Badeprodukte, Reinigungsprodukte, Shampoos, Conditioner, kombinierte Shampoos/Conditioner, Schäume, Styling-Gele, Haarsprays, Haarfärbungen, Haarfärbeprodukte, Haarbleiche, Dauerwellenprodukte, Haarentkräuselungspräparate, Nagellack, Nagellackentferner, Nagelcremes und -lotionen, Nagelhautweichmacher, Sonnencreme, Insektenschutzmittel, Anti-Aging-Produkte, Lippenstifte, Foundations, Gesichtspuder, Eye-Liner, Lidschatten, Rouges, Make-up, Mascaras, feuchtigkeitsspendende Präparate, Foundations, Körper- und Handpräparate, Hautschutzpräparate, Gesichts- und Nackenpräparate, Tonika, Kompressen, Haarpflegemittel, Aerosolfixiermittel, Duftstoffzubereitungen, Aftershaves, Make-up-Präparate, Soft Focus-Anwendungen, Nacht- und Tag-Hautpflegepräparate, nicht-färbende Haarpräparate, Bräunungspräparate, synthetische und nicht-synthetische Seifenstücke, Handflüssigkeiten, Nasenstreifen, nicht-gewebte Anwendungen zur Körperpflege, Baby-Lotionen, Babybäder und -Shampoos, Baby-Conditioner, Rasur-Präparate, Gurkenscheiben, Haut-Pads, Make-up-Entferner, Gesichtsreinigungsprodukte, Kühlcremes, Sonnencremeprodukte, Schäume, Spritze, pastöse Masken und Schlämme, Gesichtsmasken, Colognes und Eau de Toilettes, Haar-Cuticula-Überzüge, Duschgele, Gesicht- und Körperwaschmittel, Rinse-off-Körperpflegeprodukte, Gele, Schaumbäder, Scheuermittel, Gesichtswasser, Nagel-Conditioner, Lidschattenstifte, Puder für Gesicht oder Augen, Lippenbalsame, Lipglosses, Haarpflegepumpsprays und andere Nicht-Aerosol-Sprays, Haar-Frizz-Kontroll-Gele, Conditioner zum Belassen im Haar, Haarpomaden, Haarentwirrende Produkte, Haarfixativa, Haarbleichungsprodukte, Hautlotionen, Vorrasur- und Vorelektrorasurmittel, wasserfreie Cremes und Lotionen, Öl/Wasser-, Wasser/Öl-, multiple und Makro- und Mikro-Emulsionen, wasserbeständige Cremes und Lotionen, Anti-Akne-Päparate, Mundspülungen, Massage-Öle, Zahnpasten, klare Gele und Stifte, Salbengrundlagen, topische Wundheilungsprodukte, Aerosol-Körperpuder, Barriere-Sprays, Vitamin- und Anti-Aging-Präparate, Kräuter-Extrakt-Präparate, Badesalze, Bade-und Körpermilche, Haarstyling-Hilfen, feste weiche Augen-, Nagel- und Hautanwendungen, kontrollierte Freigabe-Körperpflegeprodukte, Haar-Conditioner-Sprays, Hautpflegefeuchtigkeits-Sprays, Hautpflegetücher, Porenhautpflegetücher, Porenreiniger, Hautunreinheitenentferner, schälende Hautpeelings, Hautschuppungsverstärker, Hauttüchlein und -tücher, Enthaarungspräparate, Körperpflegegleitmittel, Nagelfärbungspräparate, Sonnenschutzmittel, Kosmetika, Haarpflegeprodukte, Hautpflegeprodukte, Zahnpasten, Drug-Delivery-Systeme zur topischen Anwendung von medizinischen Zusammensetzungen, die auf die Haut aufzutragen sind, umfasst werden.

14. Eine Pestizidzusammensetzung, eine Düngerzusammensetzung, eine Düngerbeschichtung, eine Saatgutbeschichtung, eine Antifouling-Beschichtung oder wasserbasierte Beschichtungsformulierung, die die Silikonzusammensetzung gemäß Anspruch 1 oder das Monomer, das gemäß Anspruch 8 erhältlich ist, umfassen.

## Revendications

1. Composition de silicone comprenant un monomère ayant au moins une liaison carbosiloxane répondant à la formule générale (I) :
(R¹R²R³)Si-Y¹-[Si(R⁴R⁵)(Y²)]ₐ-Si(R⁶R⁷)-Z (I)
où a va jusqu'à environ 100 ;
Y¹ représente un groupe de liaison alkyle divalent substitué ou non substitué de 1 à environ 10 atome(s) de carbone ;
Y² représente un oxygène ;
R¹, R², R³, R⁴, R⁵, R⁶ et R⁷ sont indépendamment choisis dans le groupe constitué par des groupes hydrocarbonés monovalents aliphatiques, cycloaliphatiques ou aromatiques ou halogénés de 1 à environ 10 atome(s) de carbone ;
Z répond à la formule générale (II) suivante
-R¹¹-B-X (II)
où R¹¹ représente un groupe de liaison alkyle divalent ramifié ayant jusqu'à environ 20 atomes de carbone ou un hydrocarbure fluoré, un groupe aralkyle ou arylalkyle, et B représente un fragment fonctionnel hydrophile divalent constitué d'hydrocarbures aliphatiques, cycloaliphatiques ou aromatiques contenant des hétéroatomes et
X représente un groupe polymérisable choisi dans le groupe constitué par des hydrocarbures aromatiques ou aliphatiques insaturés substitués ou non substitués, des acrylates et des méthacrylates, ou où R¹¹ représente un groupe de liaison alkyle divalent linéaire ayant jusqu'à environ 20 atomes de carbone, ou un hydrocarbure fluoré, un groupe aralkyle ou arylalkyle, B représente un fragment fonctionnel hydrophile ou hydrophobe divalent choisi dans le groupe des fragments divalents suivants
-O-(C₂H₄O)ₚ-(C3H₆O)_{q}-(C₄H₈O)ᵣ-
où p et q valent indépendamment 0 à environ 100 ; r vaut 0 à environ 50 et (p + q + r) est supérieur à 0, 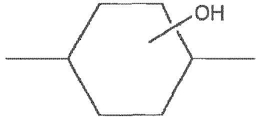 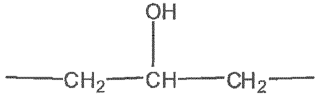 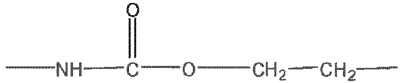 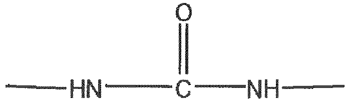 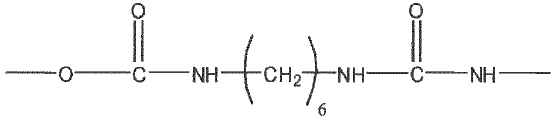 et X représente un groupe polymérisable choisi dans le groupe constitué par des hydrocarbures aromatiques ou aliphatiques insaturés, substitués ou non substitués, des acrylates et des méthacrylates.

2. Composition de silicone de la revendication 1, dans laquelle chacun de R¹, R², R³, R⁴, R⁵, R⁶, R⁷, comprend indépendamment un groupe hydrocarboné monovalent saturé de 1 à environ 9 atome(s) de carbone, un hydrocarbure fluoré, un groupe aralkyle ou arylalkyle, et R¹¹ comprend indépendamment un groupe hydrocarboné divalent saturé ayant jusqu'à environ 9 atomes de carbone.

3. Composition de silicone de la revendication 1, dans laquelle B est choisi dans le groupe constitué par l'un des fragments divalents suivants :
-O-(C₂H₄O)ₚ-(C₃H₆O)_{q}-(C₄H₈O)ᵣ- où p et q valent indépendamment 0 à environ 100 ; r vaut 0 à environ 50 et (p + q + r) est supérieur à 0, 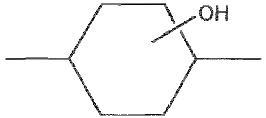 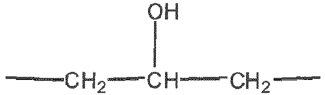 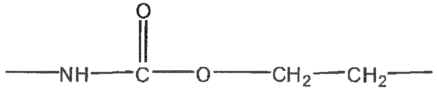 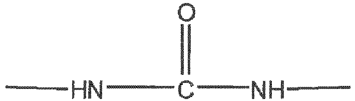 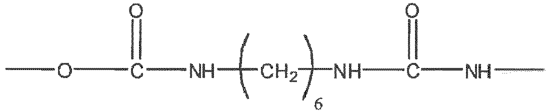 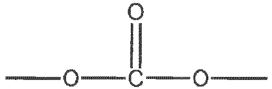 et

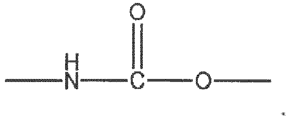

4. Composition de silicone de la revendication 1, dans laquelle X représente un fragment polymérisable répondant à la formule générale (III) 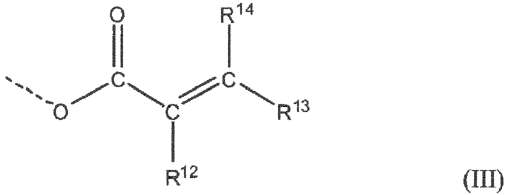 où R¹², R¹³ et R¹⁴ représentent un hydrogène ou un groupe hydrocarboné halogéné ou hydrocarboné monovalent saturé substitué ou non substitué de 1 à environ 20 atome(s) de carbone.

5. Composition de la revendication 1, comprenant un polyéthylène glycol hydrophile et répondant à la formule générale montrée ci-dessous : 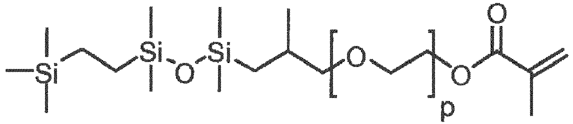 où p est supérieur à 5.

6. Composition de la revendication 1 répondant à la formule

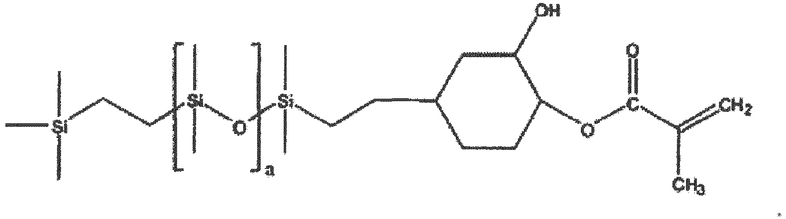

7. Composition de silicone de la revendication 1, dans laquelle l'un de R¹ à R¹⁴ comprend un hydrocarbure fluoré ou
dans laquelle chacun de R¹ à R¹⁴ comprend indépendamment au moins un fragment choisi dans le groupe constitué par des hydrocarbures aromatiques ou aliphatiques monovalents saturés ayant 1 à environ 9 atome(s) de carbone, ou
dans laquelle B comprend au moins un groupe fonctionnel constitué par des alcools, des éthers, des esters, des amides, des aminés, des acides et leurs sels, un cyano, un thio, un uréthane, l'urée, le carbonate, le carbamate, des sulfonates, des sulfonamides et des phosphates.

8. Procédé de fabrication du monomère répondant à la formule (I) de la revendication 1, comprenant la réaction d'un composé contenant de la silicone répondant à la formule générale (IV) :
(R¹R²R³)Si-Y¹-[Si(R⁴R⁵)(Y²)]ₐ-Si(R⁶R⁷)-H (IV)
où a va jusqu'à 100 ;
Y¹ représente un groupe de liaison alkyle divalent substitué ou non substitué de 1 à 10 atome(s) de carbone ;
Y² représente un oxygène ;
R¹, R², R³, R⁴, R⁵, R⁶ et R⁷ sont indépendamment choisis dans le groupe constitué par des groupes hydrocarbonés monovalents aliphatiques, cycloaliphatiques ou aromatiques ou halogénés de 1 à environ 10 atome(s) de carbone ;
avec un groupe à insaturation terminale répondant à la formule générale (V) :
R¹⁵-B-M (V)
où R¹⁵ représente un groupe alkyle insaturé ramifié ayant jusqu'à environ 20 atomes de carbone, B représente un fragment fonctionnel hydrophile divalent constitué par des hydrocarbures aliphatiques, cycloaliphatiques ou aromatiques et des hydrocarbures aliphatiques, cycloaliphatiques ou aromatiques substitués ou non substitués, saturés ou non saturés contenant des hétéroatomes,
où B comprend des fonctionnalités choisies dans le groupe constitué par un alkyle, un alcool, un éther, un ester, un amide, une amine, un acide et ses sels, un cyano, un thio, un uréthane, l'urée, le carbonate, le carbamate, un sulfonate, un sulfonamide, un phosphate et leurs combinaisons ;
M est choisi dans le groupe constitué par des groupes hydroxyle, halogène, époxy et acide carboxylique pour produire un carbosiloxane fonctionnalisé ;
et la réaction du carbosiloxane fonctionnalisé avec un composé alkylacryloyle répondant à la formule (VI) suivante : 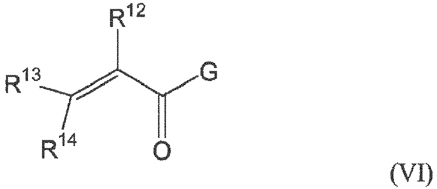 où G est choisi dans le groupe constitué par un halogène, un hydroxyle et un alkyloxy ayant 1 à environ 10 atome(s) de carbone et R¹², R¹³ et R¹⁴ sont indépendamment choisis dans le groupe constitué par un hydrogène, des hydrocarbures monovalents saturés substitués ayant 1 à environ 20 atome(s) de carbone et des hydrocarbures monovalents saturés non substitués ayant 1 à environ 20 atome(s) de carbone pour produire ledit monomère de silicone répondant à la formule générale (I) de la revendication 1.

9. Procédé de la revendication 8, dans lequel ladite réaction dudit carbosiloxane fonctionnalisé en une quantité qui se trouve dans la plage allant d'environ 5 à environ 90 pour cent en poids de la composition totale avec ledit composé alkylacryloyle en une quantité qui se trouve dans la plage allant d'environ 5 à environ 80 pour cent en poids de la composition totale est réalisée en présence d'au moins une base tertiaire ou d'au moins une résine échangeuse d'ions (IER) et d'un solvant polaire à bas point d'ébullition.

10. Composition de soin de santé comprenant la composition de silicone de la revendication 1 ou le monomère pouvant être obtenu par le procédé de la revendication 8, qui comprend en outre des bioactifs, des agents antiacnéiques, des agents antivieillissement, des agents anticaries, des agents antifongiques, des agents antimicrobiens, des antioxydants, des agents anticancer, des antiviraux, des anti-inflammatoires, des anticoagulants, des agents hémostatiques, des exfoliants, des hormones, des enzymes, des composés médicinaux, des biocides, des analgésiques externes, des agents de soin bucco-dentaires, des médicaments de soin bucco-dentaires, des agents oxydants, des agents réducteurs, des agents protecteurs de la peau, des huiles essentielles, des insectifuges, des agents absorbant la lumière UV, des filtres solaires, des pigments, des agents hydratants, des vitamines et leurs combinaisons.

11. Produit de soin de santé comprenant la composition de silicone de la revendication 1 ou le monomère pouvant être obtenu par le procédé de la revendication 8, dans lequel des hydrogels, des systèmes d'administration de médicament, des timbres transdermiques, des timbres cicatrisants, des pansements, des bouchons de punctum, des spirales ophtha, des implants rétiniens, l'iontophorèse transdermique, des échafaudages pour génie tissulaire, des dispositifs antimicrobiens, des dispositifs antibactériens, des revêtements résistants à la formation de biofilms, des dispositifs antifongiques, des dispositifs de gestion de la plaie, des dispositifs ophtalmiques, des bioinserts, des prothèses et des implants corporels sont compris.

12. Composition de soin personnel comprenant la composition de silicone de la revendication 1 ou le monomère pouvant être obtenu par le procédé de la revendication 8, qui comprend en outre des tensioactifs, des émulsifiants, des solvants, des émollients, des hydratants, des humectants, des pigments, des colorants, des parfums, des biocides, des agents de conservation, des agents chélateurs, des antioxydants, des agents antimicrobiens, des agents antifongiques, des agents antisudorifiques, des exfoliants, des hormones, des enzymes, des composés médicinaux, des vitamines, des acides alpha-hydroxy, des acides bêta-hydroxy, des rétinols, la niacinamide, des éclaircissants pour la peau, des sels, des électrolytes, des alcools, des polyols, des agents absorbants le rayonnement ultraviolet, des extraits botaniques, des huiles organiques, des cires, des agents épaississants, des charges particulaires, des silicones, des argiles, des plastifiants, des agents occlusifs, des exhausteurs sensoriels, des esters, des résines, des filmogènes, des émulsifiants filmogènes, des matériaux à indice de réfraction élevé et leurs combinaisons.

13. Produit de soin personnel comprenant la composition de silicone de la revendication 1 ou le monomère pouvant être obtenu par le procédé de la revendication 8, dans lequel des produits antisudorifiques/désodorisants, y compris des sprays, des produits en bâton ou avec applicateur à bille, des produits de rasage, des lotions pour la peau, des hydratants, des lotions toniques, des produits pour le bain, des produits de nettoyage, des shampoings, des après-shampoing, des shampooings/après-shampoing combinés, des mousses, des gels de coiffage, des pulvérisateurs capillaires, des teintures capillaires, des produits colorés pour cheveux, des produits de blanchiment capillaires, des produits bouclants, des produits lissants, des vernis à ongles, un dissolvant pour vernis à ongles, des crèmes et lotions pour les ongles, des produits pour ramollir les cuticules, un écran solaire, des insectifuges, des produits antivieillissement, des rouges à lèvres, des fonds de teint, des poudres pour le visage, des eyeliners, des fards à paupières, des blushes, des produits de maquillage, des mascaras, des préparations hydratantes, des fonds de teint, des préparations pour le corps et pour les mains, des préparations de soin cutané, des préparations pour le visage et le cou, des toniques, des pansements, des produits d'aide au coiffage, des fixateurs en aérosol, des préparations de parfum, des lotions après-rasage, des préparations de maquillage, des applications à effet « soft focus », des préparations de soin cutané pour le jour et la nuit, des préparations non colorantes pour les cheveux, des préparations bronzantes, des pains de savon synthétiques et non synthétiques, des liquides pour les mains, des bandelettes pour le nez, des applications non tissées pour le soin personnel, des lotions pour bébé, des produits lavants et shampooings pour bébés, des après-shampooing pour bébés, des préparations pour le rasage, des tranches de concombre, des tampons démaquillants, des produits démaquillants, des produits de nettoyage pour le visage, des cold creams, des produits de protection solaire, des mousses, des rafraîchisseurs de boucles, des masques en pâte et boues, des masques pour le visage, des eaux de cologne et des eaux de toilette, des produits de protection de la cuticule des cheveux, des gels-douche, des produits lavants visage et corps, des produits de soin personnel à rincer, des gels, des bains moussants, des gommages, des produits astringents, des produits traitants pour les ongles, des ombres à paupière en bâtons, des poudres pour le visage et les yeux, des baumes à lèvres, des brillants à lèvres, des produits de soin capillaire en flacon-pompe et autres pulvérisateurs non aérosol, des gels anti-frisottis, des après-shampooing sans rinçage, des pommades pour les cheveux, des produits démêlants, des produits fixants pour les cheveux, des produits de blanchiment des cheveux, des lotions pour la peau, des produits avant-rasage et avant rasage électrique, des crèmes et lotions anhydres, des émulsions huile/eau, eau/huile, multiples et macro et micro, des crèmes et lotions résistant à l'eau, des préparations antiacné, des bains de bouche, des huiles de massage, des dentifrices, des gels et sticks transparents, des bases pour pommades, des produits cicatrisants topiques, des talcs en aérosol, des pulvérisateurs barrières, des préparations vitaminées et antivieillissement, des préparations aux extraits de plantes, des sels de bain, des laits pour le bain et pour le corps, des produits d'aide au coiffage, des applications solides pour les cheveux, les yeux, les ongles et qui adoucissent la peau, des produits de soin personnel à libération contrôlée, des brumes après-shampooing, des brumes hydratante de soin pour la peau, des lingettes pour la peau, des lingettes pour les pores, des produits nettoyants pour les pores, des anticernes, des produits exfoliants pour la peau, des produits favorisant la desquamation de la peau, des serviettes et gants pour la peau, des préparations dépilatoires, des lubrifiants de soin personnel, des préparations colorantes pour les ongles, des écrans solaires, des cosmétiques, des produits de soin pour les cheveux, des produits de soin pour la peau, des dentifrices, des systèmes d'administration de médicament pour l'application topique de compostions médicinales qui doivent être appliquées sur la peau sont compris.

14. Formulation pesticide, formulation d'engrais, formulation d'enrobage d'engrais, d'enrobage de semences, de revêtement antisalissure ou de revêtement à base d'eau comprenant la composition de silicone de la revendication 1 ou le monomère pouvant être obtenu par le procédé de la revendication 8.
